# EUROPÄISCHE PATENTSCHRIFT

(11) **EP 0 716 689 B1**
(45) Veröffentlichungstag und Bekanntmachung des Hinweises auf die Patenterteilung: **02.10.2002**
(21) Anmeldenummer: 95925778.3
(22) Anmeldetag: 28.06.1995
(51) Int. Cl.: C12N 5/00, C12N 5/08, A61K 35/28

(54) **VERFAHREN ZUR HERSTELLUNG UND ZÜCHTUNG HÄMATOPOETISCHER VORLÄUFERZELLEN**
PROCESS FOR PRODUCING AND BREEDING HAEMATOPOETIC PRECURSOR CELLS
PROCEDE DE PRODUCTION ET DE CULTURE DE CELLULES PRECURSEURS HEMATOPOIETIQUES

(30) Priorität: 30.06.1994 DE 4422667
(43) Veröffentlichungstag der Anmeldung: 19.06.1996
(73) Patentinhaber: Boehringer Ingelheim International GmbH, 55218 Ingelheim am Rhein (DE)
(72) Erfinder: BEUG, Hartmut, A-1140 Wien (AT); WESSELY, Oliver, A-1180 Wien (AT); STEINLEIN, Peter, A-1020 Wien (AT); DEINER, Eva, A-1030 Wien (AT); VON LINDERN, Maartje, Marie, A-1100 Wien (AT)
(86) Internationale Anmeldenummer: EP9502516
(87) Internationale Veröffentlichungsnummer: WO96000777

(56) Entgegenhaltungen:
- WO-A-93/18136
- WO-A-93/25216
- WO-A-94/08039
- EMBO JOURNAL, Bd. 12, Nr. 3, 1993 EYNSHAM, OXFORD GB, Seiten 951-960, SCHROEDER, C. ET AL 'The estrogen receptor cooperates with the TGF-alpha receptor (c-erbB) in regulation of chicken erythroid progenitor self-renewal' in der Anmeldung erwähnt
- CELL, Bd. 74, 1993 NA US, Seiten 157-169, HAYMAN M.J. ET AL 'Self-renewal and differentiation of normal avian erythroid progenitor cells: Regulatory roles of the TGF-alpha/c-ErbB and SCF/c-Kit receptors' in der Anmeldung erwähnt
- http://www.ncbi.nlm.nih.gov:80/entrez/query.fcg- i?CMD=search&DB=PubMed, retrieved on 25.04.2000, & BIOCHEM BIOPHYS RES COMMUN, VOL 270(3) 1029-1035, 04.2000

## Beschreibung

Die vorliegende Erfindung bezieht sich auf ein Verfahren zur in vitro Herstellung und zur *in vitro* Züchtung von hämatopoetischen Vorläuferzellen der erythroiden Entwicklungsreihe.

Während der normalen Hämatopoese entwickeln sich pluripotente Stammzellen in Vorläuferzellen, die auf eine bestimmte Entwicklungsreihe festgelegt sind (diese Vorläufer werden als "committed" bezeichnet); von diesen Zellen wird angenommen, daß sie sich von den pluripotenten Stammzellen in zweierlei Hinsicht unterscheiden: Erstens sind sie in ihrer Fähigkeit, in eine einzige oder in eine geringe Anzahl spezifischer Entwicklungsreihen zu differenzieren, beschränkt. Zweitens sind solche "committed" Vorläuferzellen nach allgemein vertretener Ansicht entweder unfähig, sich ohne gleichzeitige Differenzierung kontinuierlich zu vermehren (diese Eigenschaft wird auch als Fähigkeit zur Selbsterneuerung bezeichnet) oder sie tun dies nur transient (Till und McCulloch, 1980). Daher wird angenommen, daß die Vorläuferzellen mit ihrer Festlegung auf eine bestimmte Entwicklungsreihe ein vorbestimmtes Programm von Veränderungen in der Genexpression beginnen, an dessen Ende die Bildung einer terminal differenzierten Zelle steht. Von pluripotenten Stammzellen wird hingegen angenommen, daß sie ihre Fähigkeit, zahlreiche Zellteilungen zu durchlaufen, beibehalten, ohne ihren Status der Differenzierung bzw. Genexpression zu ändern. Das Programm, das die Vorläuferzellen durchlaufen, ist selbstverständlich mit dem Durchlaufen zahlreicher Zellteilungen kompatibel, wobei jedoch angenommen wird, daß die Zellen während jeder Teilung Änderungen, wenn auch vielleicht geringfügige, ihres Status der Differenzierung bzw. Genexpression durchmachen (Keller, 1992).

Diese Ansicht, daß ein fixes Determinierungs/Differenzierungsprogramm die Entwicklung der "committed" Vorläuferzellen bestimmt, wurde kürzlich verschiedentlich in Frage gestellt: Erstens lassen einige Beobachtungen vermuten, daß normale "committed" Vorläufer verlängerte Phasen der Expansion durchlaufen können, was auf eine Selbsterneuerung oder damit verwandte Vorgänge hinweist. Murine B-Lymphozytenvorläufer erneuern sich anhaltend unter einer Reihe von Kulturbedingungen (stromale Fütterungszellschichten plus Interleukin 7), differenzieren aber unter anderen Bedingungen in reife B-Zellen (Rolink et al., 1991). Auf ähnliche Weise können einzelne murine Granulozyten-Makrophagenkolonien bildende Zellen (GM-CFC) in Abhängigkeit von der Konzentration von GM-CSF zwischen 100 und mehr als 10.000 reife Granulozyten und Makrophagen produzieren (Metcalf, 1980).

Ein weiteres Phänomen, das mit einem fixen Programm der Entwicklung von "committed" Vorläufern schwer vereinbar ist, sind Leukämien. Obwohl diese in einigen Fällen von pluripotenten Stammzellen ausgehen, leiten sich andere Leukämien eindeutig von "committed" Vorläufern ab (Sawyers et al., 1991). Hinsichtlich des letzteren Typs gibt es ein wiederholt geäußertes Konzept, daß die in Leukämiezellen ablaufenden genetischen Veränderungen diesen die abnormale Fähigkeit zur Selbsterneuerung verleihen, eine Eigenschaft, die die entsprechende normale Vorläuferzelle nicht hat. Während in der chronischen Phase der chronisch myeloischen Leukämie (CML) Klone von veränderten, multipotenten Vorläuferzellen die entsprechenden normalen Klone überwachsen (vielleicht aufgrund ihrer verstärkten Fähigkeit zur Selbsterneuerung), führen weitere Mutationen, die während der Blastenkrise stattfinden, zu einem massiven Auswachsen von unreifen Vorläufern und reifenden Zellen von einer speziellen Entwicklungsreihe, was als Selbsterneuerung von abnormalen "committed" Vorläufern interpretiert wird (Daley et al., 1990; Elefanty et al., 1990; Kelliher et al., 1990).

Kürzlich wurde von Hühnerzellen gezeigt, daß normale hämatopoetische Vorläufer, die auf die erythroide Entwicklungsreihe festgelegt sind, unter spezifischen Bedingungen zur anhaltenden Selbsterneuerung fähig sind (Schroeder et al., 1993; Hayman et al., 1993). Dabei wurde gezeigt, daß die kombinierte Wirkung von TGFα ("Transforming Growth Factor", ein Ligand für das Hühnerhomologe des epidermalen Wachstumsfaktorrezeptors/c-erbB-Protoonkogens (TGFαR/cerbB; Lax et al., 1988) und Östradiol das Auswachsen von normalen Vorläufern aus Hühnerknochenmark induzierte. Diese Zellen werden aufgrund ihrer Eigenschaft, aus Kulturen auszuwachsen, die TGFα plus Östradiol oder SCF (Stammzellfaktor; Stem Cell Factor) enthalten, als "SCF/TGFα-Vorläuferzellen" bezeichnet (Zellen, die in Gegenwart von SCF wachsen, werden als "SCF-Vorläuferzellen" bezeichnet). SCF/TGFα-Vorläuferzellen exprimieren das c-kit-Protoonkogen, den Östradiolrezeptor und TGFαR/c-erbB und sind in Gegenwart von TGFα plus Östradiol bis zum Ende ihrer normalen *in vitro*-Lebensdauer zur anhaltenden Selbsterneuerung fähig. Es wurde auch gezeigt, daß erythroide Vorläufer, die von normalen CFU-Es ("colony-forming unit erythroids") hinsichtlich aller untersuchten Eigenschaften nicht unterscheidbar sind (als SCF-Vorläufer bezeichnet), aus Knochenmark mittels Hühner-SCF herausgezüchtet werden konnten (Hayman et al., 1993). Im Gegensatz zu den SCF/TGFα-Vorläufern mit der Fähigkeit zur Selbsterneuerung fehlte den SCF-Vorläufern die Expression von TGFαR/c-erbB, und die Zellen zeigten in Gegenwart von SCF nur eine transiente Selbsterneuerung während der Dauer von 7 bis 10 Tagen. Wenn auf Differenzierungsfaktoren umgeschaltet wurde (Erythropoetin plus Insulin), differenzierten beide Typen mit voneinander nicht unterscheidbarer Kinetik in Erythrozyten. Das deutete darauf hin, daß die SCF/TGFα-Vorläufer nicht die Vorläufer von SCF-Vorläufern sind, wie ursprünglich aufgrund der Tatsache angenommen wurde, daß SCF/TGFα-Vorläufer relativ selten sind
(1 von 15.000 normalen Knochenmarkszellen), während die SCF-Vorläufer viel häufiger sind (1 von 300-500; Hayman et al., 1993). Diese Ergebnisse ließen jedoch die Frage offen, ob die selbsterneuernden SCF/TGFα-Vorläufer von noch unreiferen Vorläufern abstammen. Eine mögliche Antwort ist die, daß diese Zellen einen gesonderten, seltenen Zelltyp darstellen, der bereits im Knochenmark vorkommt und sich aus multipotenten Vorläufern ähnlich wie eine gesonderte Abstammungslinie entwickelt. Eine alternative Antwort wäre, daß diese Zellen von normalen CFU-Es abstammen, die das Potential zur Selbsterneuerung nur unter der Wirkung von spezifischen Kombinationen von Wachstumsfaktoren und Hormonen erwerben, die normalerweise bei der Erythropoese nicht aktiv sind.

In früheren Arbeiten (Schroeder et al., 1993) wurde gezeigt, daß für das Auswachsen von SCF/TGFα-Vorläufern aus Knochenmark zwei grundsätzliche Anforderungen bestehen: Erstens eine bestimmte Zeitdauer - das Auswachsen fand nie vor Ablauf von 11 bis 14 Tagen statt; zweitens die Abhängigkeit von sowohl TGFα als auch Östradiol, was sich dadurch zeigte, daß das Auswachsen der Zellen durch einen Östradiolantagonisten komplett inhibiert wurde und bei Fehlen von TGFα nicht auftrat. Falls die erste Antwort richtig ist und SCF/TGFα-Vorläufer ein eigener Zelltyp sind, der stets in normalem Knochenmark vorhanden und nur von TGFα und Östradiol abhängig ist, sollten sich andere Faktoren auf die Häufigkeit dieser Zellen nicht wesentlich auswirken; gegen dieses vereinfachte Modell sprechen jedoch zwei Beobachtungen: erstens wurde gefunden, daß das Auswachsen von SCF/TGFα-Vorläufern in Gegenwart von Hühnerserum, das mit Tierkohle behandelt worden war, stark inhibiert wurde, während es in Freon-behandeltem oder unbehandeltem Serum nicht wesentlich beeinträchtigt wurde. Dies ließ vermuten, daß neben TGFα und Östradiol andere Faktoren, die durch die Tierkohlebehandlung entfernt werden, eine Auswirkung auf die SCF/TGFα-Vorläufer in irgendeinem Stadium ihrer Entstehung haben. Außerdem wurde beobachtet, daß Knochenmarkszellen, die in SCF plus Östradiol gehalten wurden, nach 8 bis 10 Tagen stationär wurden, jedoch ungefähr am 14. Tag wieder ein langsames Wachstum aufnahmen. Diese Zellen exprimierten TGFαR/c-erbB in relativ hoher Konzentration (Hayman et al., 1993) und konnten in TGFα plus Östradiol gezüchtet werden, was vermuten läßt, daß diese Zellen SCF/TGFα-Vorläufer sind, die aus der ursprünglichen Population von SCF-Vorläufern herausgewachsen waren.

Der vorliegenden Erfindung lag die Aufgabe zugrunde, die Mechanismen aufzuklären, die an der Bildung von hämatopoetischen Vorläufern der erythroiden Entwicklungsreihe, die c-Kit und TGFαR/c-ErbB exprimieren (im Rahmen der vorliegenden Erfindung als "SCF/TGFα-Vorläufer" bezeichnet), beteiligt sind und auf der Grundlage der gewonnenen Erkenntnisse ein Verfahren bereitzustellen, das die Kultivierung von normalen erythroiden Vorläufer-Zellen *in vitro* erlaubt.

Insbesondere sollte ein Verfahren bereitgestellt werden, das die massenhafte Herstellung von nichtimmortalisierten und somit genetisch nicht veränderten humanen hämatopoetischen Vorläuferzellen ermöglicht.

Im Rahmen der vorliegenden Erfindung wurde zunächst für Hühnerzellen gezeigt, daß sich in Gegenwart von SCF, TGFα, Östradiol und bestimmten, nicht identifizierten Hühnerserumfaktoren SCF/TGFα-Vorläufer in Kulturen von gereinigten SCF-Vorläufern entwickeln.

Es wurde gezeigt, daß bei Kultivierung von SCF-Vorläufern in Gegenwart einer Kombination von SCF, TGFα, Östradiol und zunächst nicht definierten Faktoren aus normalem oder anämischem Hühnerserum ein großer Anteil dieser Zellen weder eine Differenzierung noch eine Apoptose durchmacht, sondern beginnt, in einem strikt zeitabhängigen Verlauf steigende Mengen von TGFαR/c-erbB zu exprimieren, was nach 10 bis 14 Tagen im Erhalt von SCF/TGFα-Vorläufern resultiert. Zu diesem Zeitpunkt ist offensichtlich die Expression von TGFαR/c-erbB in den Zellen hoch genug, um die Proliferation in Gegenwart von TGFα und Östradiol bei Abwesenheit von SCF zu ermöglichen. Bei Verwendung von speziell behandelten Hühnerseren konnte gezeigt werden, daß keine SCF/TGFα-Vorläufer gebildet werden, wenn einer dieser drei Faktoren (SCF, TGFα oder Östradiol) fehlte. Andererseits wurde die Bildung der proliferierenden Vorläufer in Gegenwart von SCF, TGFα und Östradiol teilweise inhibiert, wenn auch nicht beseitigt, wenn die nicht identifizierte Aktivität des Hühnerserums fehlte. In weiteren Versuchen am Hühnermodell konnte gezeigt werden, daß die zunächst nicht identifizierte Aktivität zumindestens teilweise durch zwei definierte Faktoren ersetzt werden kann: 1. den Glucocorticoidrezeptor-Liganden Dexamethason und 2. den Tyrosinkinase-Rezeptor-Liganden Insulin-like Growth factor 1 (IGF-1). Es konnte ferner angenommen werden, daß Erythropoietin ein weiterer Faktor ist, der für die Aktivität im Hühnerserum verantwortlich ist.

Wenn SCF-Vorläufer in SCF, TGFα und Östradiol gezüchtet wurden, reicherten sich SCF/TGFα-Vorläufer in der Kultur an, bis nach ca. zwei bis zweieinhalb Wochen diese in der Kultur vorherrschten. Die Expression von TGFαR/c-erbB nahm mit der Zeit zu, wenn die SCF-Vorläufer in SCF, TGFα und Östradiol gezüchtet wurden. Aufgrund der durchgeführten Massenkulturexperimente konnte zunächst nicht zwischen zwei Möglichkeiten unterschieden werden, auf welche Weise die SCF/TGFα-Vorläufer aus den Kulturen von SCF-Vorläufern entstanden sind. Die erste (triviale) Möglichkeit wäre, daß eine kleine Anzahl von SCF/TGFα-Vorläufern, die c-erbB exprimieren, in normalem Knochenmark und somit in SCF-Vorläuferpopulationen von vornherein existiert, wobei diese Zellen, (wenn die Kultur in Gegenwart von SCF, TGFα und Östradiol durchgeführt wird, was diesen Zellen möglicherweise zu einem Wachstumsvorteil verhilft), allmählich die SCF-Vorläufer überwachsen. Die nicht triviale, interessantere Möglichkeit wäre jedoch, daß im Knochenmark von vornherein keine erythroiden Vorläufer vorhanden sind, die in TGFα und Östradiol allein proliferieren können, sondern daß SCF/TGFα-Vorläufer zur Entwicklung aus SCF-Vorläufern induziert werden, wenn alle drei Faktoren plus bestimmte Hühnerserumkomponenten bzw. Dexamethason und IGF-1, (siehe oben) vorhanden sind.

Die im Rahmen der vorliegenden Erfindung durchgeführten Experimente zeigten, daß die letztere Hypothese zutrifft, daß sich also SCF/TGFα-Vorläufer aus SCF-Vorläufern entwickeln können. Die mit Hühnerzellen erhaltenen Resultate ergaben, daß normale erythroide Vorläufer (SCF-Vorläufer, die in allen untersuchten Eigenschaften dem "Colony Forming Unit Erythroid"(CFU-E)-Vorläufer ähneln), sich unter der Kontrolle von wenigstens zwei Wachstumsfaktoren (SCF, TGFα) plus einem Steroidhormon (Östradiol) und einer zunächst nicht definierten Aktivität aus Hühnerserum, die später teilweise identifiziert wurde als Dexamethason plus IGF-1, zu einem anderen Typ von erythroidem Vorläufer (SCF/TGFα-Vorläufer) entwickeln. Dieser andere Vorläufertyp ist durch seine neu erworbene Expression von TGFαR/c-ErbB, (der dem Säugetier-EGF/TGFα Rezeptor entspricht), und seiner Fähigkeit, als Reaktion auf TGFα und Östradiol eine verlängerte Selbsterneuerung zu durchlaufen, gekennzeichnet. Das Differenzierungsprogramm der SCF/TGFα-Vorläufer nach Behandlung mit Differenzierungsfaktoren (EPO, Insulin) ähnelt stark dem normaler CFU-E-Vorläufer (Hayman et al., 1993).

Da von erythroiden Vorläufern bisher angenommen wurde, daß sie irreversibel auf Differenzierung festgelegt sind, wobei sie ein fixes Programm von 5 bis 10 Zellteilungen durchmachen, war der mit Hühnerzellen erhaltene Befund, daß diese unter bestimmten Bedingungen durch eine Änderung ihres Differenzierungsprogramms ("developmental switch") ein Selbsterneuerungspotential erlangen können, von großem Interesse, vorausgesetzt, daß diese Befunde für Säugetier- oder sogar humane Zellen Gültigkeit haben würden. Im Rahmen der vorliegenden Erfindung wird gezeigt, daß die Ergebnisse des Hühnersystems in überraschendem Ausmaß auf menschliche Zellen anwendbar sind.

Bedarf an humanen hämatopoetisehen Vorläuferzellen, die *in vitro* kultiviert werden können, besteht vor allem im Hinblick auf die Transplantation solcher Zellen bei der Behandlung von Krebs- und AIDS-Patienten.
Eine weitere Anwendung einer solchen Tranaplantation ist die gentherapeutische Behandlung von chronischen Anämien, bei denen die Reifung der Erythrozyten gestört ist, z.B. Thalassämien und andere genetisch bedingte Anämien.

Eine der wenigen als definitiv angesehenen Voraussetzungen, die für die erfolgreiche Transplantierbarkeit von Blutzellen erforderlich sind, ist die Expression von CD34. Es ist jedoch nicht bekannt, in welchem Entwicklungsstadium sich die Subpopulation der CD34⁺-Zellen befindet, die für die erfolgreiche Transplantation tatsächlich verantwortlich ist, wenn auch angenommen wird, daß Entwicklungsreihe und Differenzierungsstadium der Zellen offensichtlich eine Rolle spielen.

Die autologe oder allogene Transplantation von hämatopoetischen Vorläuferzellen ist mit Schwierigkeiten verbunden, wobei eines der Hauptprobleme darin besteht, daß eine ausreichende Zahl von Zellen mit dem für die erfolgreiche Rekonstitution des hämatopoetischen Systems erforderlichen Proliferationspotential transplantiert werden muß und die Kriterien, die dieses Potential bestimmen, noch nicht ausreichend erforscht sind.

Bisher wurden für allogene Transplantationen häufig Knochenmarkzellen von gesunden Spendern verwendet; für autologe Transplantationen werden Stammzellen aus peripherem Blut, die während der Erholung des Patienten von der Chemotherapie und/oder durch Behandlung mit rekombinanten Wachstumsfaktoren mobilisiert werden, eingesetzt. Diese Methoden sind aufwendig; außerdem sind sie für den Spender mit großen Unannehmlichkeiten verbunden bzw. liefern aufgrund von hämatologischen Veränderungen des Patienten geringe Ausbeuten. Es wurde daher kürzlich als Alternative vorgeschlagen, Stammzellen von zytokinbehandelten gesunden Spendern einzusetzen.

Eine als vielversprechend angesehene Alternative besteht ferner darin, Nabelschnurblutzellen statt Knochenmark- oder CD34⁺-positiven Zellen aus peripherem Blut zu verwenden, weil die Mehrheit der hämatopoetischen Stamm- und Vorläuferzellen von Nabelschnurblut sich in einem früheren Entwicklungsstadium befindet und ein größeres Proliferationspotential besitzt. Da jedoch bei einem Erfordernis von 5 x 10⁵ - 2 x 10⁶ CD34⁺-Zellen pro kg Körpergewicht für die Transplantation eines Erwachsenen ca. 1.5 l Nabelschnurblut erforderlich wären, stößt diese Methode bei der Behandlung von Erwachsenen auf Grenzen.

Es besteht daher Bedarf an einem Verfahren, das die Massenkultur von autolog oder allogen transplantierbaren hämatopoetischen Vorläuferzellen ermöglicht.

Im Rahmen der vorliegenden Erfindung wurde gezeigt, daß humane erythroide Vorläuferzellen überraschenderweise ein ähnliches Verhalten zeigen wie die entsprechenden Hühnerzellen, indem sie eine Änderung in ihrem Differenzierungsprogramm durchmachen, aufgrund derer sie ein Selbsterneuerungspotential erwerben. Ebenso wie die Hühnerzellen benötigten die Zellen SCF, Östradiol und Dexamethason, um die Fähigkeit zur langandauernden Selbsterneuerung zu erwerben. IGF-1 beeinflußte das Wachstum von Hühner- und menschlichen Erythroblasten positiv. Einige der Zellen in der Kultur humaner erythroider Vorläufer, die im Rahmen der durchgeführten Versuche erhalten wurde, reagierten auf Liganden des EGF-Rezeptors, was ein zusätzlicher Hinweis darauf ist, daß die humanen Zellen in ihrem Verhalten unter dem Einfluß bestimmter Wachstums- und Differenzierungsfaktoren den Hühner-SCF/TGFα-Vorläufern ähnlich sind.

Die vorliegende Erfindung beruht somit ferner auf der entscheidenden Erkenntnis, daß eine Änderung im Differenzierungsprogramm humaner erythroider Vorläufer auftreten muß, aufgrund welcher Änderung sie die Fähigkeit zum anhaltenden Auswachsen erlangen. Diese Änderung des Differenzierungsprogramms sollte durch das Zusammenwirken von Faktoren induziert werden, welche Liganden von Vertretern derselben Rezeptorgruppen sind, deren Aktivierung die Entwicklung von selbsterneuernden erythroiden Vorläufern aus Hühnerknochenmark induziert.

Die Erfindung betrifft somit ein Verfahren zur *in vitro* Herstellung von nichtimmortalisierten hämatopoetischen Vorläuferzellen der erythroiden Entwicklungsreihe, dadurch gekennzeichnet, daß man Zellen, enthaltend eine Population erythroider Vorläufer, in einem Medium, das die für das Wachstum erythroider Zellen erforderlichen üblichen Komponenten enthält, zumindest solange mit einer Kombination aus Wachstumsfaktoren, enthaltend
a) mindestens einen Liganden des Östrogenrezeptors, ausgewählt aus natürlichen oder synthetischen Steroidhormonen, die den Östrogenrezeptor wie Östradiol aktivieren,
b) mindestens einen Liganden des Glucocorticoid-Rezeptors, ausgewählt aus natürlichen oder synthetischen Steroidhormonen, die den Glucocorticoidrezeptor wie Hydrocortison aktivieren, und
c) mindestens einen Liganden eines Tyrosinkinase-Rezeptors, der diesen Rezeptor aktiviert, behandelt, bis die Zellen sich selbst zu erneuern beginnen, und daß man gegebenenfalls anschließend die Zellen in einem Medium weiterzüchtet, welches die für die anhaltende Selbsterneuerung erforderlichen Faktoren enthält.

Durch die Behandlung der Zellen mit der Kombination aus Wachstumsfaktoren (im folgenden als "Faktorkombination" bezeichnet) machen die Zellen eine Änderung des Differenzierungsprogramms durch. Diese geht einher mit einer Änderung des Expressionsmusters der Rezeptoren, die durch die Wirkung der Faktorkombination neu exprimiert bzw. hochreguliert werden, und/oder durch Änderung des Expressionsmusters von Proteinkomponenten der durch diese epigenetische(n) Änderung(en) ausgelösten zellulären Signalübertragungswege.

Unter "Selbsterneuerung" wird die Fähigkeit von Zellen verstanden, Tochterzellen zu bilden, die während der nachfolgenden Zellteilungen nicht meßbar reifen, d.h. in denen keine meßbare weitere Anhäufung von solchen Proteinen stattfindet, die typisch für die reifen Zellen sind, aber in geringen Mengen auch in Vorläuferzellen exprimiert sein können. Ein weiteres wichtiges Kriterium für Selbsterneuerung ist, daß sich das Verhältnis von Proteinen der reifen (terminal differenzierten) Zelle (z.B. Hämoglobin) und Proteinen, die für die Funktion jeder Zelle notwendig sind (sog. "housekeeping proteins", z.B. glykolytische Enzyme) nicht meßbar ändert.

Bevorzugt wird das erfindungsgemäße Verfahren auf humane Zellen angewendet.

Als Ausgangszellmaterial wird vorzugsweise eine auf CD34-positive Zellen angereicherte Zellpopulation aus

Knochenmark, peripherem Blut oder, in einer besonders bevorzugten Ausführungsform, aus Nabelschnurblut verwendet. Die Anreicherung kann nach literaturbekannten Methoden erfolgen; eine Übersicht solcher Methoden wird in dem Handbuch "Hematopoietic Stem Cells, The Mulhouse Manual", 1994, gegeben.

Die Zellen werden *in vitro* zumindest so lange gezüchtet, bis ihre Selbsterneuerung eintritt. Rein äußerlich sind Zellen mit Selbsterneuerungspotential daran erkennbar, daß sie sich während einer Zeitspanne, die der in vitro Lebensdauer der Zellen (50 - 70 Generationen bei menschlichen Zellen) bzw. einem Teil dieser Lebensdauer entspricht, kontinuierlich in Kultur teilen, d.h. exponentiell proliferieren, sowie eine konstante Größe und einen vergleichsweise niedrigen Gehalt an Erythrozytenproteinen (z.B. Hämoglobin) aufweisen. Der Fachmann kann in Vorversuchen anhand dieser Kriterien feststellen, zu welchem Zeitpunkt die Zellen ein Selbsterneuerungspotential erlangt haben und dementsprechend die Dauer der Kultivierung definieren.

Das Selbsterneuerung der im Rahmen der vorliegenden Erfindung erhältlichen humanen Zellen der erythroiden Entwicklungsreihe zeichnet sich dadurch aus, daß sich die Zellen ohne erkennbare Differenzierung über einen wesentlich längeren Zeitraum teilen, als bisher für normale humane BFU-Es (burst forming unit erythroids) gezeigt wurde.

Die Faktorkombination ist vorzugsweise eine Kombination aus mindestens drei, vorzugsweise mindestens vier Faktoren, wobei mindestens zwei davon Liganden von Tyrosinkinase-Rezeptoren sind. Über Rezeptoren dieses Typs, ihre Zugehörigkeit zu Familien und Subfamilien, ihre Liganden sowie die durch ihre Aktivierung ausgelösten Signalübertragungswege existiert eine Fülle von Literatur, es werden laufend neue Vertreter identifiziert. Den Tyrosinkinase-Rezeptoren ist gemeinsam, daß sie nach Bindung ihres Liganden sich selbst an Tyrosinen phosphorylieren. Nach dieser Autophosphorylierung interagieren die Phosphotyrosinreste mit spezifischen zytoplasmatischen Molekülen, wodurch die zelluläre Antwort auf die Wachstumsfaktoren ausgelöst wird.

Die Familie der Tyrosinkinaserezeptoren wird in verschiedene Klassen und Subfamilien eingeteilt; dazu zählen die Klasse, der die EGFR-Familie, HER2/neu/cerbB-2 und HER3/c-erbB-3 angehören; die Klasse, der der Insulin-Rezeptor, der "Insulin Related Receptor" und der IGF-1-Rezeptor angehören; die Klasse, umfassend PDGF-Rezeptor, PDGFß-Rezeptor, MCSF-1-Rezeptor und c-kit; die Klasse der Fibroblast Growth Factor Receptors (FGF-Rezeptorl, FGF-Rezeptor2, FGF-Rezeptor3, FGF-Rezeptor4) und der HGFR-Rezeptor (Hepatocyte Growth Factor Receptor). Einige dieser Klassen haben das Merkmal gemeinsam, daß die Kinasedomäne von einer Sequenz unterbrochen ist. Bezüglich der Tyrosinkinase-Rezeptoren und ihren Liganden wird auf die Übersichtsartikel von Fantl et al., 1993, und Van der Geer, 1994, einschließlich der darin zu den einzelnen Rezeptoren spezifisch zitierten Literatur Bezug genommen.

Die Faktorkombination aus Tyrosinkinase-Rezeptor-Liganden besteht aus mindestens je einem Liganden für Rezeptoren aus verschiedenen Familien innerhalb der Tyrosinkinase-Rezeptoren. Ein Beispiel für eine solche Kombination ist
i) mindestens ein Ligand eines Tyrosinkinase-Rezeptors, der eine durchgehende Kinasedomäne besitzt, und
ii) mindestens ein Ligand eines Tyrosinkinase-Rezeptors, der eine von einem Insert unterbrochene Kinasedomäne besitzt.

Beispiele für Vertreter der in i) definierten Rezeptoren sind die Mitglieder der EGF-Rezeptorfamilie (Human Epidermal Growth Factor Receptor 1 - 4); zu dieser Familie zählen weitere, erst teilweise identifizierte Rezeptoren.

Liganden der in i) definierten Rezeptoren sind u.a. EGF, TGFα, NDF (Neuronal Differentiation Factor; Peles und Yarden, 1993), einschließlich der durch differentielles Splicing entstehenden Varianten, Heregulin, Amphiregulin, Glial Growth Factor etc. (Fantl et al., 1993).

Liganden der in ii) definierten Rezeptoren sind u.a. der c-Kit-Ligand SCF (Stern Cell Factor), Platelet Derived Growth Factor (PDGF) alpha und beta, alle Mitglieder der Fibroblast Growth Factor Familie, CSF-1 (Colony Stimulating Factor 1), und vaskularisierede Faktoren (z.B. VEGF, Vascular Endothelial Growth Factor) (Fantl et al. 1993).

Daneben existiert eine Vielzahl von nicht eindeutig einer der dieser beiden Gruppen zuzurechnenden Tyrosinkinase-Rezeptoren (deren Liganden erst zum Teil bekannt sind), deren Aktivierung durch die entsprechenden Liganden das Auswachsen menschlicher Vorläuferzellen bewirken kann; die entsprechenden Liganden können ebenfalls im Rahmen der vorliegenden Erfindung eingesetzt werden. Zu diesen Rezeptoren zählen: Hepatocyte Growth Factor Receptor (dessen Ligand auch als "Scatter factor" bezeichnet wird; die von Galimi et al., 1994, erhaltenen Befunde weisen darauf hin, daß der Hepatocyte Growth Factor Receptor (HGFR), von dem angenommen wird, daß der dieselben Signalübertragungswege aktiviert wie der EGF-Rezeptor, in CD34+ Zellen und daraus entstehenden humanen erythroiden Vorläuferzellen eine wichtige Rolle spielt), c-sea und c-ros (deren Liganden noch nicht identifiziert sind), diverse epithelzellspezifische Rezeptoren, deren Liganden unbekannt sind, einer Gruppe von kürzlich beschriebenen (u.a. von Tamagnone et al., 1993 und Kaipainen et al., 1993), aus erythroiden Zellen klonierten Rezeptoren, deren Liganden ebenfalls noch unbekannt sind, sowie die Mitglieder der Neurothrophinrezeptoren (trk, trk-B, trk-C mit den Liganden NGF, BNDF etc.), ferner Rezeptoren der Insulin-Rezeptor Familie (Insulin-Rezeptor, IGF-1-Rezeptor etc.).

Ohne auf die Theorie festgelegt sein zu wollen, dürfte es für die Auslösung der Änderung des Differenzierungsprogramms wesentlich sein, daß durch die Bindung der Liganden und die dadurch bewirkte Aktivierung der in i) und ii) definierten Rezeptoren unterschiedliche Signalübertragungswege in Gang gesetzt werden.

Neben den beiden Liganden der Tyrosinkinase-Rezeptoren enthält die Faktorkombination
iii) mindestens einen Liganden des Östrogen- und mindestens einen Liganden des Glucocorticoid-Rezeptors.

Geeignet sind im Rahmen der vorliegenden Erfindung natürliche oder synthetisch hergestellte Steroidhormone, die wie Östradiol den Östrogenrezeptor bzw, wie Hydrocortison den Glucocorticoidrezeptor aktivieren.

Daneben kann die Faktorkombination gegebenenfalls Liganden des Progesteronrezeptors, wie Aldosterol und Progesteron, enthalten.

Gemeinsam ist diesen Hormonen, daß sie a) niedermolekular sind, daß sie b) an im Kern lokalisierte Rezeptoren binden, die durch das Hormon in ihrer Aktivität regulierte Transkriptionsfaktoren (Proteine, die Gene in ihrer Aktivität verändern) darstellen und daß sie c) in einigen der bisher untersuchten Systeme das Differenzierungsprogramm von Zellen verändern können.

Im Rahmen der vorliegenden Erfindung hat sich gezeigt, daß neben einem Östrogen vor allem Dexamethason, ein Glucocorticoid, von entscheidender Bedeutung für das Auswachsen sich selbst erneuernder Hühner- und humaner erythroider Vorläuferzellen ist.

Ferner kann die Faktorkombination
iv) einen oder mehrere zusätzliche Faktoren enthalten.

Als zusätzliche Faktoren iv) kommen vor allem Liganden in Betracht, die zumindest die Beschleunigung der Änderung des Differenzierungsprogramms und damit ein effizienteres Auswachsen der Zellen bewirken. Diese Faktoren werden im allgemeinen bereits am Beginn der Kultur- dem Medium beigegeben, wobei zu berücksichtigen ist, daß verschiedene Faktoren zu verschiedenen Zeitpunkten während der Änderung des Differenzierungsprogramms erforderlich sein können.

Im Hinblick auf die Beschleunigung der Änderung des Differenzierungsprogramms kann es daher zweckmäßig sein, Faktoren, die für die Auslösung dieses Vorganges erforderlich, später jedoch gegebenenfalls entbehrlich bzw. sogar nachteilig sind, zu einem geeigneten Zeitpunkt, der durch Serienversuche ermittelt werden kann, aus dem Medium zu entfernen. Als zusätzliche Faktoren kommen in Betracht:
1. Liganden von Rezeptoren, die über Serinphosphorylierung von Zielproteinen wirken. (TGFβ-Rezeptor Familie). Hier sind vor allem die auch in der frühen Embryonalentwicklung eine Rolle spielenden Liganden Activin, Inhibin, BMP etc. (Laufer, 1993; Hogan, 1993) von Bedeutung.
2. Liganden weiterer Tyrosinkinase-Rezeptoren, insbesondere IGF-1 oder Hepatocyte Growth Factor (HGF).
3. Vertreter der großen Gruppe der Zytokine bzw. Interleukine (Wachstums- und Differenzierungsfaktoren im hämatopoetischen- und Immunsystem). Beinahe alle diese Zytokine binden an Rezeptoren, die selbst keine bekannte Enzymaktivität besitzen, einige der Rezeptoren bilden jedoch mit intrazellulären Tyrosinkinasen Komplexe. Eine Übersicht über diese ständig wachsende Familie von Rezeptoren und deren Liganden ist in Boulay and Paul, 1993, gegeben.

Wesentlich für die Wirkung eines im Rahmen der vorliegenden Erfindung anwendbaren Zytokins ist, daß es erstens die Proliferation unreifer Vorläufer stimuliert und zweitens keine Wirkung besitzt, die das Zellwachstum negativ beeinflußt und/oder Apoptose (programmierten Zelltod) auslöst. Im Rahmen der vorliegenden Erfindung bevorzugte Zytokine sind IL-1, IL-3, IL-11, IL-13. Besonders bevorzugt ist EPO.

Die durch Einwirkung der Faktorkombination erhaltene Population von Zellen kann nach Beginn der Selbsterneuerung eingefroren und bei Bedarf aufgetaut und danach entweder weitergezüchtet oder direkt transplantiert werden, um das in vitro erworbene Selbsterneuerungspotential der Zellen für die Proliferation *in vivo* zu nützen.

Die Zellen können jedoch über die Zeitspanne hinaus, während der sie das Selbsterneuerungspotential erwerben, kultiviert werden, um innerhalb der Population eine größere Zahl von proliferierenden Zellen zu erhalten.

Die Weiterzüchtung der proliferierenden Zellen wird in Gegenwart derjenigen Wachstums- und Differenzierungsfaktoren durchgeführt, die die Zellen für die anhaltende Selbsterneuerung benötigen.

Für Hühnerzellen ist TGFα einer der Faktoren, die erforderlich sind für das anhaltendende Selbterneuerungspotential und somit für die Kultivierung der Zellen über einen längeren Zeitraum im Hinblick auf den Erhalt einer großen Zahl von Zellen. Für humane Zellen sind bevorzugt eingesetzte Faktoren für die Weiterzüchtung der Zellen Liganden des unter i) definierten Typs, wie EGF und/oder TGFα, und/oder HGF, sowie SCF, ferner EPO und IGF-1.

Die geeignete Faktorkombination sowohl für die Induktion der Selbsterneuerung als auch für die Weiterzüchtung der proliferierenden Zellen wird ermittelt, indem das Ansprechen der Zellen und ihr Wachstumsverhalten unter Einwirkung verschiedener Faktormischungen zu verschiedenen Zeitpunkten getestet wird; Beispiele für derartige Tests sind u.a. in den Beispielen 4 b), 5, 7 b, 8 dargestellt. Dabei wird die Faktormischung zweckmäßig derart optimiert, daß zunächst verschiedene Mehrkomponentenmischungen getestet werden, um die am besten wirksame Mischung zu identifizieren. Danach wird aus der am besten wirksamen Mischung Schritt für Schritt jeweils ein Faktor entfernt und das Verhalten der Kultur mit und ohne den Faktor verglichen. Insgesamt wird die Faktorkombination darauf optimiert, mit möglichst wenigen Faktoren ein möglichst schnelles und effizientes Auswachsen zur Selbsterneuerung fähiger Zellen zu erreichen.

Die im Rahmen der vorliegenden Erfindung durchgeführte Behandlung mit der Kombination aus SCF, TGFα, Östradiol und einer weiteren Aktivität bewirkte in Hühner- und humanen Zellen eine Zunahme der Expression von biologisch aktivem TGFαR/c-ErbB, die sich in Hühnerzellen auch durch eine Zunahme an nach Ligandenzugabe autophosporyliertem Rezeptor manifestierte; die weitere Aktivität war im Fall von Hühnerzellen eine nicht näher identifizierte Aktivität im Hühnerserum und im Fall von Humanzellen EPO.

In einer Ausführungsform der Erfindung besteht die Faktorkombination für die Herstellung humaner hämatopoetischer Vorläuferzellen aus
i) einem Liganden eines Rezeptors aus der Familie der EGF-Rezeptoren und/oder des HGF-Rezeptors,
ii) einem Liganden von c-Kit;
iii) Östradiol und Dexamethason, und
iv) Erythropoetin und IGF-1.

In einer besonderen Ausführungsform der Erfindung ist
i) EGF und/oder TGFα und/oder HGF, und
ii) SCF.

Sofern die Faktoren in ausreichender Konzentration Bestandteile des Mediums sind, z.B. als Serumkomponenten, müssen sie nicht gesondert zugesetzt werden.

Die neben der Faktorkombination im Medium enthaltenen für das Wachstum der Zellen erforderlichen üblichen Komponenten, wie Vitamine, Aminosäuren, etc., sind dem Fachmann geläufig; sie sind in kommerziell erhältlichen Medien enthalten bzw. können einschlägigen Handbüchern, wie "Hematopoietic Stem Cells, The Mulhouse Manual", 1994, und Fachartikeln, wie Sawada et al., 1990, entnommen werden.

Die nach dem erfindungsgemäßen Verfahren erhaltenen Zellen können, nach Entfernung des Kulturmediums, in einem für die therapeutische Anwendung geeigneten Medium, z.B. in Humanserumalbumin (HSA) oder autologem Plasma, suspendiert und für die allogene bzw. autologe Transplantation verwendet werden. Das erfindungsgemäße Verfahren kann u.a. eingesetzt werden, um aus einem Vorrat von Blutzellen eines Individuums, dessen Produktion CD34-positiver Zellen, z.B. durch Behandlung mit Zytokinen, angeregt wurde, im Fall des Erfordernisses einer Transplantation hämatopoetische Zellen heranzuzüchten. Diese kann eingefroren gelagert werden, bei Bedarf aufgetaut, durch in vitro Kultur amplifiziert und gegebenenfalls nach geeignetem Gentransfer, für therapeutische Zwecken am Patienten eingesetzt werden.

Ein Beispiel für eine Strategie, bei der genetisch veränderte, in vitro kultivierte menschliche erythroide Zellen zum Einsatz kommen können, ist die Behandlung der Sichelzell-Anämie durch Gentherapie. Diese Erbkrankheit kommt vor allem in den USA in einer großen Zahl von farbigen Patienten vor. Eine mögliche Vorgehensweise besteht im Anzüchten von erythroiden Vorläufern aus Knochenmark, peripherem Blut oder (bei pränataler Diagnosestellung) Nabelschnurblut, Gentransfer des die "hereditary persistence of foetal haemoglobin" (HPFH) Mutation tragenden, humanen Globin-Gen Locus und Applikation dieser genetisch veränderten somatischen Zellen die Keimbahn ist nicht betroffen) beim Patienten.

Der Gentransfer in die erfindungsgmäß erhältlichen Zellen kann mit Hilfe von Standardmethoden für die Transfektion derartiger Zellen erfolgen. Dazu zählen der Gentransfer mittels viraler Vektoren (Retrovirus, Adenovirus, Adeno-assoziiertes Virus) oder mittels nicht-viralen Systemen auf Basis der Rezeptorvermittelten Endozytose; Übersichten über gebräuchliche Methoden werden z.B. von Mitani und Caskey, 1993; Jolly, 1994; Vile und Russel, 1994; Tepper und Mule, 1994; Zatloukal et al., 1993, WO 93/07283 gegeben.

### Figurenübersicht

- Fig. 1:: Auswachsen von SCF/TGFα-Vorläufern aus SCF-Vorläufern
- Fig. 2:: Expression und Bioaktivität von c-Kit und c-ErbB während der Proliferation von SCF-Vorläufern in SCF, TGFα und Östradiol
- Fig. 3:: Änderung der Wachstumsfaktorabhängigkeit von SCF-Vorläufern, die in SCF, TGFα und Östradiol proliferieren
- Fig. 4:: Versuchsstrategie zur Klärung der Entstehung von SCF/TGFα-Vorläufern
- Fig. 5:: Entwicklung von SCF/TGFα-Vorläufern aus SCF-Vorläufern
- Fig. 6A,B:: In SCF, TGFα und Östradiol gewachsene LD-Klone entsprechen SCF/TGFα-Vorläufern: Expression von bioaktivem c-ErbB und Proliferationsreaktion auf TGFα
- Fig. 7A,B:: Ein Faktor in Hühnerserum erleichtert die Entwicklung von SCF-Vorläufern zu SCF/TGFα-Vorläufern
- Fig. 8A:: Erfordernis von Östradiol und SCF während der Entwicklung von SCF/TGFα-Vorläufern aus SCF-Vorläufern
- Fig. 8B:: Beschleunigung der Entwicklung von SCF- zu SCF/TGFα-Vorläufern durch anämisches Hühnerserum
- Fig. 9A:: Beschleunigung der Umwandlung von SCF-Vorläufern zu SCF/TGFa Vorläufern durch Dexamethason
- Fig. 9B:: Definition von Insulin-like Growth Factor (IGF-1) als einer der für die Aktivität im Hühnerserum verantwortlichen Faktoren
- Fig. 10:: Auswachsen von erythroiden Zellen aus humanen CD34⁺-Zellen aus peripherem Blut
- Fig. 11A,B:: Charakterisierung von *in vitro* kultivierten humanen erythroiden Vorläufern
- Fig. 12A:: Verlängerung des Selbsterneuerungs-Potentials menschlicher CD34⁺-Zellen aus Nabelschnurblut durch Dexamethason
- Fig. 12B:: Steigerung des Wachstums menschlicher Zellen aus peripherem Blut durch IGF-1
- Fig. 13:: Vergleich der Eigenschaften von auswachsenden menschlichen erythroiden Vorläufern mit Hühnerzellen

In den folgenden Beispielen wurden, wenn nicht anders angegeben, die von Hayman et al., 1993, beschriebenen Materialien und Methoden verwendet. In den Beispielen 1-6 wurden Hühnerzellen verwendet, in den Beispielen 7 und 8 humane Zellen.

### Beispiel 1

### Anreicherung von SCF/TGFα-Vorläufern durch Kultivierung von SCF-Vorläufern in SCF, TGFα und

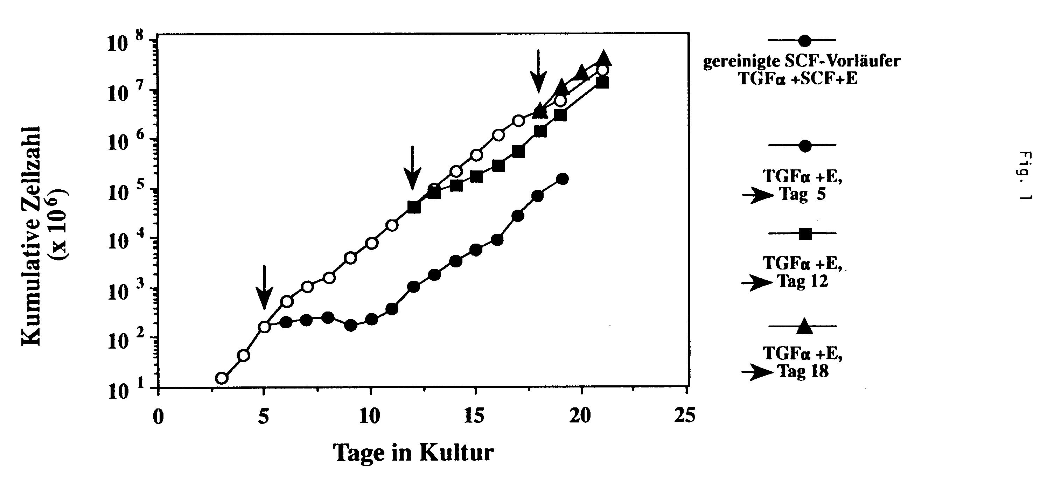

Um systematisch festzustellen, ob SCF-Vorläufer SCF/TGFα-Vorläufer enthalten oder ob sie Zellen enthalten, die sich zu diesem Zelltyp entwickeln können, wurden Zellen aus einer 6 Tage alten Kultur von gereinigten SCF-Vorläufern (Hayman et al., 1993) in CFU-E-Medium, enthaltend 100 ng/ml rekombinanten SCF, 5 ng/ml TGFα und 5 x 10⁷ M Östradiol, ausgesät und die Zellproliferation mittels Zählung nach dem von Hayman et al., 1993, beschriebenen System (CASY-1, Schärfe System) überwacht. (Der Verwendung aller drei Faktoren lag die Überlegung zugrunde, die SCF-Vorläufer solange wie möglich am Leben zu erhalten und gleichzeitig das Wachstum etwaiger in der Kultur von vornherein vorhandener oder im Laufe der Kultur gebildeter SCF/TGFα-Vorläufer zu stimulieren.) Das Ergebnis dieses Versuchs ist in Fig. 1 dargestellt. Überraschenderweise (und in starkem Gegensatz zu den in einem Vergleichsversuch erhaltenen Ergebnissen aus der Züchtung derselben Zellen in TGFα plus Östradiol) zeigten die Zellen nur eine schwache, vorübergehende Abnahme in ihrer Wachstumsrate um die Tage 8 bis 10, proliferierten jedoch im Anschluß daran exponentiell weiter, wobei von Tag 25 bis 30 die Verdoppelungszeit 18 bis 22 h betrug, worauf Zellalterung eintrat (Fig. 1, offene Kreise).

Um festzustellen, ob die SCF-Vorläufer, die in SCF, TGFα und Östradiol gezüchtet worden waren, SCF/TGFα-Vorläufer enthielten oder sich solche entwickelt hatten, und um deren Häufigkeit grob abzuschätzen, wurden Aliquots der Kultur zu verschiedenen Zeitpunkten entnommen, gewaschen, in CFU-E-Medium, enthaltend TGFα und Östradiol, aber kein SCF, überführt und die Zellzahl mit der der Kultur verglichen, die alle drei Faktoren enthielt. (Die Zellen wurden durch entsprechende Verdünnung mit frischem Medium bei einer Dichte zwischen 1 x 10⁶ und 2 x 10⁶ pro ml gehalten und die kumulative Zellzahlen aus den erhaltenen Zellzahlen und den entsprechenden Verdünnungsfaktoren berechnet (Schroeder et al., 1993; Hayman et al., 1993)) (Fig. 1, Pfeile). Wenn den Zellen am Tag 5 die Dreifaktor-Kombination entzogen wurde, hörten sie sofort zu proliferieren auf. Die Zellzahlen blieben bis zum Tag 11 annähernd konstant. Während dieser Zeit machten die meisten Zellen eine Apoptose durch (diese wurden vom Zellzähler nicht von den lebenden Zellen unterschieden), während einige gesunde Klumpen übrig blieben, welche um den Tag 13 bis 14 die Kultur zu bilden begannen. Im Anschluß daran durchliefen die Zellen in Gegenwart von TGFα plus Östradiol ein Wachstum, das in seiner Kinetik von dem der Kontrollkultur nicht unterscheidbar war (Fig. 1, gefüllte Kreise).

Ein unterschiedliches Verhalten der Zellen wurde beobachtet, wenn Zellen nach Züchtung in SCF, TGFα und Östradiol am Tag 12 in Medium mit TGFα plus Östradiol überführt wurden (Fig. 1, gefüllte Vierecke). Bis zu diesem Zeitpunkt hatte nur ein Teil der Zellen Apoptose erlitten, während viele andere weiterwuchsen, was sich als vorübergehende Verringerung der Wachstumsrate zwischen den Tagen 13 und 16 äußerte. Danach wuchsen die Zellen mit ähnlicher Geschwindigkeit in TGFα plus Östradiol wie die Kontrollzellen. Nach 18 Tagen Kultur hatte eine Überführung von allen drei Faktoren auf TGFα plus Östradiol keinen merkbaren Effekt auf die Zellproliferation (Fig. 1, gefüllte Dreiecke), was darauf hinweist, daß zu diesem Zeitpunkt die Kultur zur Gänze aus SCF/TGFα-Vorläufern bestand.

### Beispiel 2

Zunahme der Expression von bioaktivem TGFαR/c-erbB in SCF-Vorläufern, die in SCF, TGFα und Östradiol gezüchtet werden

### a) Expression und Bioaktivität von c-Kit und c-ErbB während der Proliferation von SCF-Vorläufern in SCF, TGFα und Östradiol

Aufgrund der in Beispiel 1 erhaltenen Ergebnisse wurde angenommen, daß die in SCF, TGFα und Östradiol gezüchteten SCF-Vorläufer entweder durch von vornherein existierende SCF/TGFα-Vorläufer überwachsen wurden oder sich zu solchen entwickelt hatten. Ziel der vorliegenden Versuche war es zu zeigen, daß die proliferierenden Zellen tatsächlich bioaktiven TGFαR/c-ErbB exprimieren, was sich in den davon zu erwartenden biochemischen Reaktionen (Autophosphorylierung) oder biologischen Reaktionen (Stimulierung der Proliferation in entsprechenden Assays) äußern sollte. Zu diesem Zweck wurden an den Tagen 6, 12 und 20 Aliquots der Kultur sowie der Kontrollkultur (siehe Beispiel 1) entnommen, gewaschen, über Nacht in Medium ohne Wachstumsfaktoren inkubiert, mit verschiedenen Faktorkombinationen 5 min lang stimuliert und, wie von Hayman et al., 1993, beschrieben, sowie für den Phosphotyrosin-Blot und den anschließenden Western Blot (unter Verwendung von anti-TGFαR- bzw. c-erbB-Antikörpern) weiterbehandelt.

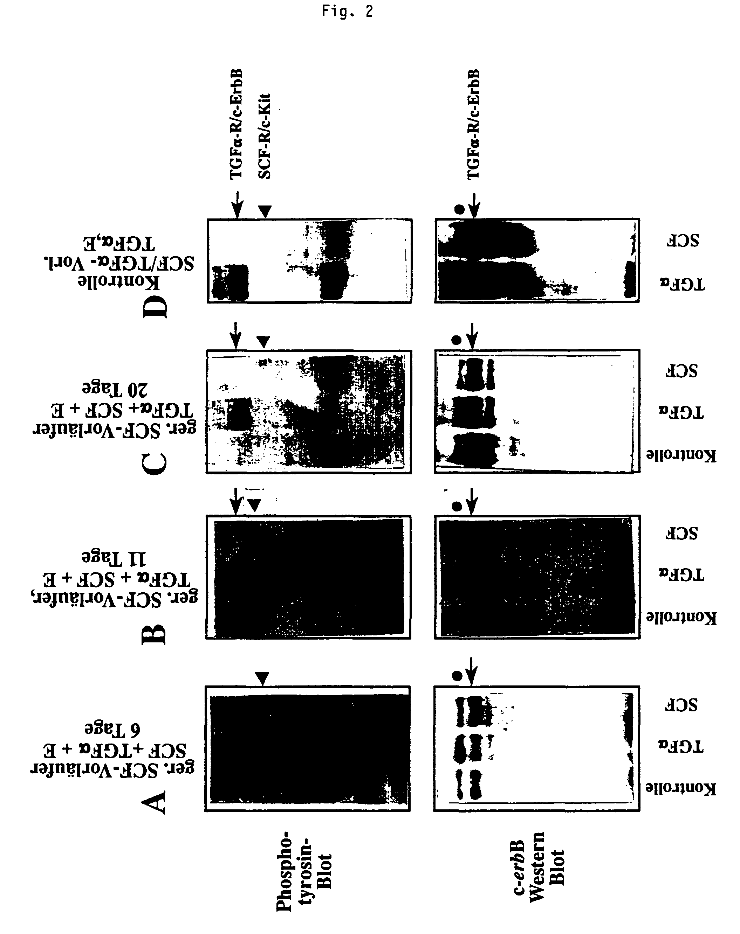

Nach insgesamt 6 Tagen (nach anfänglichen 3 Tagen in SCF) zeigten die in SCF, TGFα und Östradiol gezüchteten Zellen die aufgrund der Reaktion auf SCF erwartete deutliche Phosphorylierung von c-Kit und exprimierten c-Kit in großen Mengen. Im Gegensatz dazu enthielten die Zellen gemäß Western Blot-Analyse nur sehr geringe Mengen von TGFαR/c-ErbB, und es war keine Autophosphorylierung von c-ErbB sichtbar (diese Versuche sind in Fig. 2A dargestellt, wobei die Pfeile das 170 kd TGFαR/c-ErbB-Protein und die Pfeilspitzen das 140 kd SCF-R/c-Kit-Protein anzeigen; der schwarze Kreis in den unteren Feldern gibt die Position einer Untergrundbande ohne Bezug zu TGFαR/c-ErbB an).

Nach 11 Tagen war die Expression von TGFαR/c-ErbB deutlich angestiegen, wie mittels c-ErbB-Western Blot nachgewiesen wurde. Zusätzlich war eine schwache, wenn auch deutlich nachweisbare Autophosphorylierungsreaktion des c-ErbB-Proteins auf den Liganden nachweisbar (Fig. 2B). Wie erwartet, exprimierten die Zellen noch immer autophosphorylierbaren c-Kit (Fig. 2B).

Auf ähnliche Weise exprimierten die nach 20 Tagen getesteten Zellen (zu diesem Zeitpunkt wuchsen sie sowohl in TGFα plus Östradiol als auch in SCF, TGFα und Östradiol) erhöhte Mengen von TGFαR/c-ErbB, welcher nun als Reaktion auf TGFα deutlich autophosphorylierbar war (Fig. 2C). Überraschenderweise exprimierten die Zellen noch immer geringere Mengen an TGFαR/c-ErbB als die Kontrollzellen, die aus unbehandeltem Knochenmark in TGFα plus Östradiol gezüchtet worden waren. (Schroeder et al., 1993; Hayman et al., 1993).
Die erhaltenen Ergebnisse zeigen, daß SCF-Vorläufer. die in TGFα, SCF und Östradiol gezüchtet werden, bereits nach 6 Tagen geringe Mengen an TGFαR/c-ErbB exprimieren und im Anschluß daran während der nächsten 8 bis 14 Tage dessen Expression kontinuierlich steigern.

### b) Änderung der Wachstumsfaktorabhängigkeit von SCF-Vorläufern, die in SCF, TGFα und Östradiol proliferieren

Um zu bestätigen, daß der biochemisch nachgewiesene TGFαR/c-ErbB tatsächlich den bioaktiven Rezeptor darstellt, wurden die Zellen zusätzlich mittels [³H]-Thymidineinbau-Assay auf ihre Reaktion auf SCF, TGFα und Östradiol getestet, wie von Hayman et al., 1993, beschrieben.

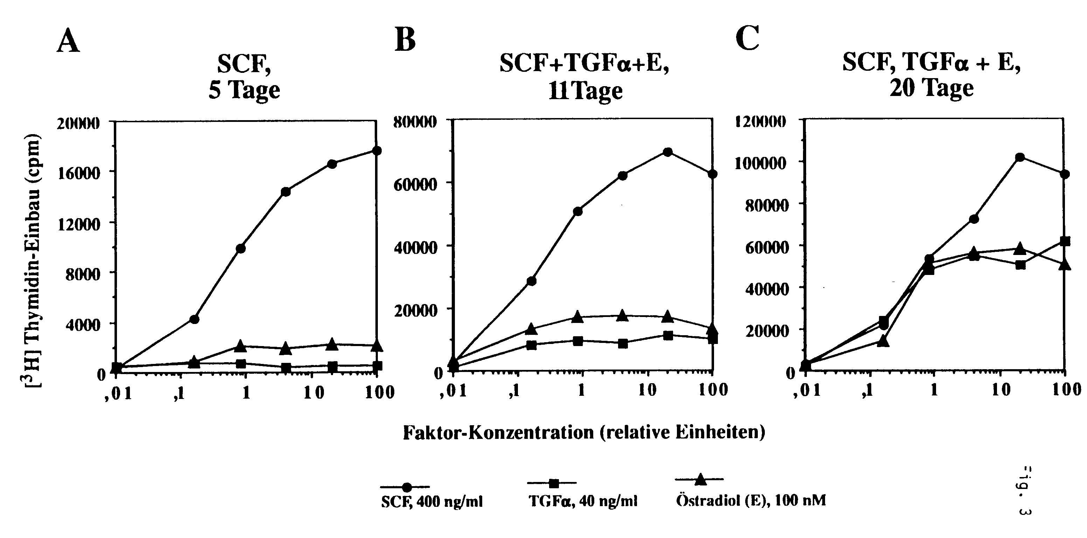

Dazu wurden Aliquots einer Kultur von SCF-Vorläufern (5 Tage, Tafel A von Fig.3) bzw. von Zellen, die 11 oder 20 Tage lang in SCF, TGFα und Östradiol gezüchtet worden waren (Tafeln B und C von Fig. 3) auf ihre Reaktion auf verschiedene Faktoren untersucht (100 relative Einheiten entsprechen den unter den Symbolen angegebenen Faktorkonzentrationen). Es zeigte sich, daß die 6 Tage lang in SCF, TGFα und Östradiol gezüchteten Zellen auf SCF und Östradiol wie erwartet reagierten, während keine Reaktion auf TGFα nachweisbar war (Fig. 3A). Nach 11 Tagen in Kontakt mit diesen Faktoren, war die Reaktion der Zellen auf SCF und Östradiol unverändert, aber nun war eine schwache, wenn auch deutliche Reaktion auf TGFα nachweisbar (Fig. 3B). Wie erwartet reagierten die 20 Tage lang in SCF, TGFα und Östradiol gezüchteten Zellen stark auf alle drei Faktoren, ohne Unterschied zu den Kontroll-SCF/TGFα-Vorläufern (Fig. 3C).

Zusammenfassend zeigen die erhaltenen Ergebnisse, daß selbsterneuernde SCF/TGFα-Vorläufer effizient aus erythroiden Vorläufern gezüchtet werden können, welche anfänglich nur auf SCF reagieren und denen sowohl nachweisbare Mengen an TGFαR/c-ErbB als auch die Fähigkeit für die länger anhaltende Selbsterneuerung fehlen.

### Beispiel 3

### Entwicklung von SCF/TGFα-Vorläufern aus SCF-Vorläufern

Um die Frage des Ursprungs von SCF/TGFα-Vorläufern aus Kulturen von SCF-Vorläufern zu klären, wurde die Methode der Klonierung mittels limitierender Verdünnung ("Limiting Dilution", im folgenden als "LD-Klonierung" bezeichnet) gewählt. Diese Methode erlaubt es, das Proliferationsverhalten (und Differenzierungsverhalten) von einzelnen proliferierenden Zellen in einer komplexen Mischung von nicht-proliferierenden Zellen zu analysieren, weil es bei geeigneter Verdünnung möglich ist, die Entwicklung einzelner proliferierender Zellen in einzelnen Vertiefungen von Zellkulturplatten (96-Well Platten) zu verfolgen. Der Erfolg einer solchen Methode hängt natürlich von einer guten Klonierungseffizienz (10 bis 50 %) der zu analysierenden proliferierenden Zellen ab, ein Kriterium, das erfüllt ist, wenn die Zahl der erhaltenen proliferierenden Klone eine lineare Funktion der Zahl an ausgesäten Zellen ist, bis zu sehr wenigen (1 bis 10) Klonen pro 96-Well Platte. (Daß dieses Kriterium für die Klärung der vorliegenden Frage erfüllt ist, wurde für SCF- und SCF/TGFα-Vorläufer von Hayman et al., 1993, gezeigt.)

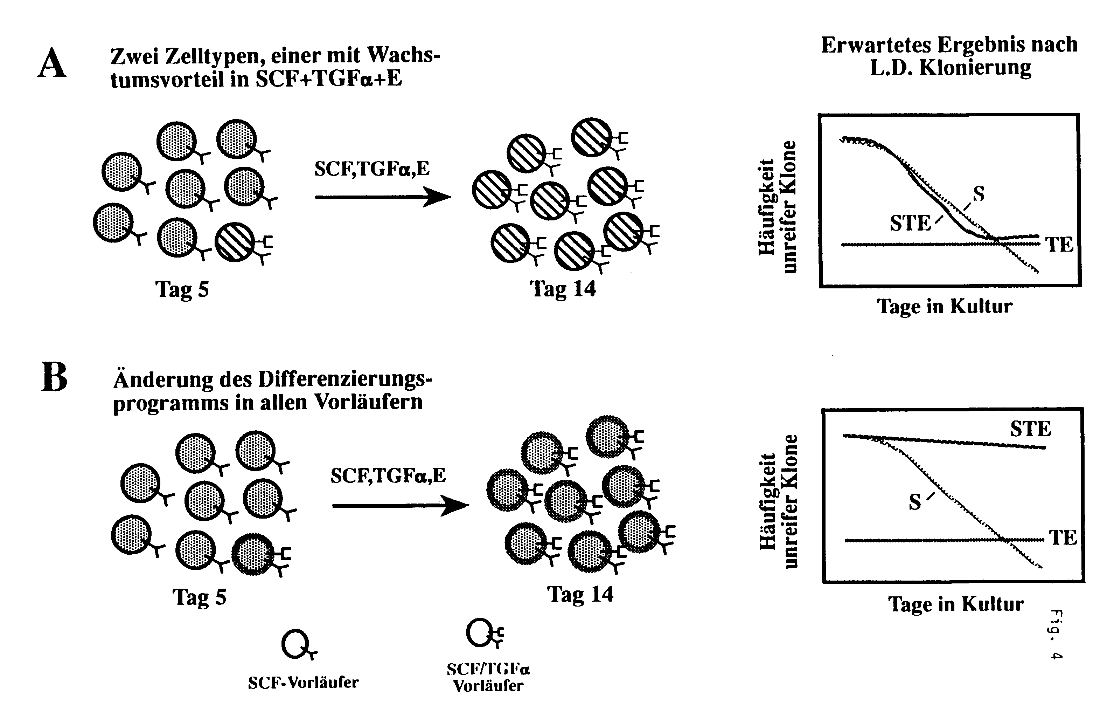

Die Überlegung, auf welche Weise die LD-Klonierung zwischen den beiden möglichen Modellen (selektives Auswachsen von seltenen SCF/TGFα-Vorläufern aus SCF-Vorläufern oder Entwicklung von SCF-Vorläufern zu SCF/TGFα-Vorläufern) ist in Fig. 4A und 4B, rechte Tafeln, dargestellt. Im linken Teil der Figur ist jeweils schematisch das Modell dargestellt, im rechten Teil der Figur das erwartete Ergebnis der LD-Klonierung, wobei Fig. 4A das Modell des selektiven Wachstumsvorteils von seltenen Vorläufern in SCF, TGFα und Östradiol und Fig. 4B das alternative Modell zeigt, dem eine Änderung des Differenzierungsprogramms vieler bzw. aller Vorläufer zugrunde liegt. Falls, entsprechend dem ersten Modell, Knochenmark sowohl seltene (einer von 20.000) SCF/TGFα-Vorläufer mit der Fähigkeit zur Selbsterneuerung und stabilen c-ErbB-Expression, als auch zusätzlich häufige (1 von 300) SCF-Vorläufer, die transient in Gegenwart von SCF proliferieren, die aber weder zu einer anhaltenden Selbsterneuerung fähig sind noch c-ErbB exprimieren, dann sollte SCF nach 4 bis 6 Tagen viele proliferierende Klone von SCF-Vorläufern induzieren. Danach sollte wegen differenzierender oder degenerierender SCF-Vorläufer die Zahl der proliferierenden Klone rasch absinken. In TGFα plus Östradiol sollte eine viel geringere Zahl von Klonen (1 von 20.000) erhalten werden, welche aufgrund der langfristigen Selbsterneuerungskapazität dieser Klone im wesentlichen konstant bleiben sollte. In Gegenwart aller drei Faktoren (SCF, TGFα und Östradiol) sollten die Kolonienzahlen anfänglich so hoch sein wie in SCF allein, sollten aber danach auf das mit TGFα plus Östradiol erhaltene Niveau absinken (Fig. 4A, rechte Tafel).

Entsprechend dem zweiten Modell enthält Knochenmark (und daher die SCF-Vorläufer) von vornherein nur wenige SCF/TGFα-Vorläufer, während sich die Mehrzahl dieser Zellen in einem langsamen Prozeß, der die Anwesenheit von SCF, TGFα, Östradiol (und Hühnerserumfaktoren) erfordert, aus SCF-Vorläufern entwickelt. Es wäre daher zu erwarten, daß die Häufigkeit der Klone, die sich bei Gegenwart aller dreier Faktoren entwickeln, mit der Zeit nicht oder nur schwach abnimmt, was im Gegensatz zu dem erwarteten Verhalten solcher Klone entsprechend dem ersten Modell steht (Fig. 4B, rechte Tafel). Die Häufigkeit der sich einerseits in Anwesenheit von SCF und andererseits in Gegenwart von TGFα und Östradiol entwickelnden Klone sollte der des ersten Modells entsprechen (Fig. 4B, rechte Tafel).

### a) LD-Klonierung gereinigter SCF-Vorläufer

Gereinigte, drei Tage alte SCF-Vorläufer wurden hergestellt, wie von Hayman et al., 1993, beschrieben. Dann wurden die Zellen in verschiedenen Konzentrationen (20 bis 2.500 Zellen pro Vertiefung der 96-Well Zellkulturplatte) in CFU-E-Medium, enthaltend entweder nur Östrogen (Kontrolle), oder nur SCF (plus den Östradiol-Antagonisten ICI 164384, um die im Serum enthaltene Östradiolaktivität zu unterdrücken), oder TGFα plus Östradiol oder SCF, TGFα und Östradiol, ausgesät. Um eine gute Klonierungseffizienz zu gewährleisten, wurden als Fütterungsschicht 50 adhärente myeloide Zellen alle Vertiefungen ausgesät. (Die myeloiden Zellen wurden erhalten, indem Knochenmarkzellen präpariert und zu 50 x 10⁶ Zellen/ml pro 100 mm Schale ausgesät und mit 10 ng/ml cMGF und SCF behandelt wurden. Während der ersten 2 bis 3 Tage wurden die nicht bzw. schwach adhärenten Zellen suspendiert und anschließend in einer größeren Schale adhärieren gelassen.) Unreife, gesunde Kolonien wurden 4, 9 und 11 Tage (entsprechend einem Gesamtalter der Zellen von 7, 12 und 14 Tagen) nach dem Aussäen der Zellen gezählt.

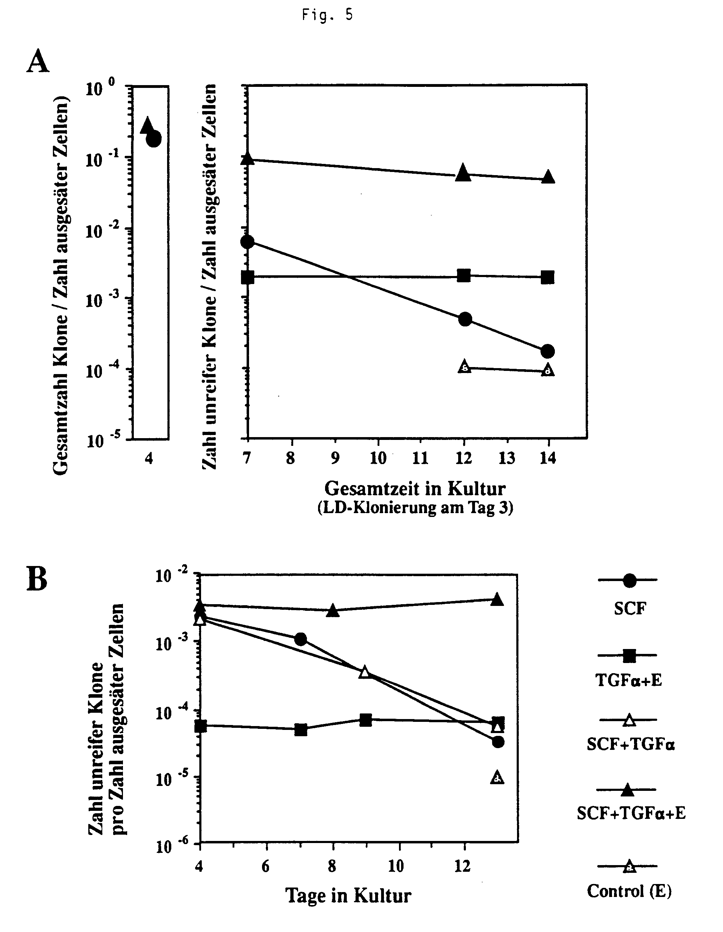

Das Ergebnis ist in Fig. 5A dargestellt (außer bei den Kontrollen in Fig. 5, wo sehr wenige Kolonien erhalten wurden, sind die festgestellten Häufigkeiten das Ergebnis der Zählung von mehr als 100 Kolonien von mindestens zwei verschiedenen Zellverdünnungen). Als Kontrolle wurde zunächst die Gesamt-Klonierungseffizienz (undifferenzierte plus differenzierte Kolonien), erhalten 2 bis 3 Tage nach dem Aussäen mit den gereinigten SCF-Vorläufern in den verschiedenen Medien, bestimmt (Fig. 5A, linke Tafel). Man sieht, daß in Gegenwart von SCF Klonierungsraten von 10 bis 20 % erhalten wurden, unabhängig von der Gegenwart von Östradiol oder TGFα. In den Medien, die TGFα plus Östradiol enthielten, oder in den nur Östradiol enthaltenden Kontrollen waren die wenigen sichtbaren Kolonien zu diesem Zeitpunkt für eine Zählung zu klein.

Aufschlußreicher waren die Ergebnisse, die mit Kolonien erhalten wurden, die mehr als 50 % gesunde, unreife Zellen enthielten. Am Tag 7 war die Zahl der in SCF allein gezüchteten Klone bereits auf < 10⁻² abgesunken, während die Klone, die in SCF, TGFα und Östradiol gezüchtet wurden, noch mit einer Häufigkeit von 10⁻¹ vorhanden waren. Die Frequenz der in TGFα plus Östradiol gewachsenen Klone war noch niedriger (2 x 10³), während die Klone in den Östradiol-Kontrollproben noch nicht sichtbar waren.

Das weitere Verhalten der in den verschiedenen Medien gewachsenen Klone stützte die Vermutung, daß sich SCF/TGFα-Vorläufer aus SCF-Vorläufern entwickeln. Unreife, in SCF allein wachsende Klone sanken zum Zeitpunkt 12 bis 14 Tage auf 3 x 10⁻⁴ bzw. 1 x 10⁻⁴ ab, wobei sie sich dem Background-Niveau (5 x 10⁻⁵) der in Östradiol allein hochgewachsenen Kolonien annäherten. Erwartungsgemäß änderte sich die geringe Zahl von Kolonien, die in TGFα plus Östrogen hochgekommen waren (2 x 10⁻³) mit der Zeit nicht. In Übereinstimmung mit der Feststellung, daß sich SCF-Vorläufer zu SCF/TGFα-Vorläufern entwickeln können (Fig. 4B), blieb ein beträchtlicher Teil der in SCF, TGFα und Östradiol gewachsenen Klone unreif und profliferationsfähig, wobei die Häufigkeit nur geringfügig abnahm (von 9 x 10⁻² am Tag 7 auf 5 x 10⁻² am Tag 14; Fig. 5A).

### b) LD-Klonierung normaler Knochenmarkszellen

Um auszuschließen, daß SCF-Vorläufer, die die Fähigkeit haben, in Gegenwart von SCF, TGFα und Östradiol ein Selbsterneuerungspotential zu erwerben, vor der LD-Klonierung durch *in vitro* Klonierung vorselektiert worden waren, wurden Versuche mit frischen, unbehandelten Knochenmarkszellen durchgeführt, um die in a) erhaltenen Ergebnisse zu bestätigen. Insbesondere sollte bestimmt werden, ob erythroide Vorläufer mit Selbsterneuerungspotential tatsächlich mit Häufigkeiten annähernd denen von SCF-Vorläufern (1 von 3.000-5.000; Hayman et al., 1993) aus einzelnen Zellen gebildet werden können, wenn sie in allen drei Faktoren gezüchtet wurden, während sie selten bleiben (1 von 15.000), wenn sie in TGFα plus Östradiol allein wachsen.

Normale Knochenmarkszellen, präpariert wie von Hayman et al., 1993, beschrieben, wurden in einem Bereich von 500 bis 15.000 Zellen pro Vertiefung in CFU-E-Medium, enthaltend verschiedene Faktorkombinationen bei 4 verschiedenen Zell-Verdünnungen (500, 2.000, 6.000, 15.000), ausgesät und zu verschiedenen Zeitpunkten nach dem Aussäen unreife Kolonien (enthaltend mehr als 50 % runde, proliferierende Zellen) gezählt. Das Ergebnis ist in Fig. 5B dargestellt: Nach 4 Tagen bildeten die in SCF gewachsenen Zellen Kolonien mit einer Häufigkeit von 3 x 10⁻² bis 5 x 10⁻². Danach nahm die Häufigkeit der unreifen Kolonien progressiv ab, wobei nach 13 Tagen eine Häufigkeit von 2 x 10⁻⁵ erreicht wurde. Dabei trat ein zunehmender Anteil von Zellen in die Differenzierung ein und machte anschließend eine Apoptose durch. Wie erwartet, waren die in TGFα plus Östradiol gewachsenen Klone von Anfang an selten (6 x 10⁻⁵ bis 8 x 10⁻⁵), die Häufigkeit blieb jedoch im wesentlichen während des Experiments konstant. Andererseits wurden die in SCF, TGFα plus Östradiol gewachsenen Klone am Tag 4, 8 und 13 mit einer Häufigkeit von 3 x 10⁻² bis 5 x 10⁻² gefunden. Es können somit die drei Faktoren SCF, TGFα und Östradiol tatsächlich das Auswachsen von unreifen Kolonien aus Knochenmark mit einer Häufigkeit induzieren, die der bei SCF-Vorläufern nach 4 Tagen entspricht und die der Häufigkeit von Zellen in normalem Hühnerknochenmark, welche CFU-E-Kolonien bilden können, ähnlich ist.

Abschließend sollte festgestellt werden, ob tatsächlich alle drei Faktoren erforderlich sind, um das Auswachsen von unreifen LD-Klonen mit hoher Häufigkeit zu induzieren. In Medien, enthaltend einzelne Faktoren (Östradiol allein, TGFα oder SCF plus ICI 164384, um das endogene Serumöstradiol zu unterdrücken) wurden nur sehr geringe Zahlen von unreifen Klonen erhalten (ca. 10⁻⁵). In TGFα plus SCF ohne Östradiol verhielten sich die Klone genauso wie in SCF allein, d.h. sie waren am Tag 4 häufig und nahmen dann progressiv ab (Fig. 5B). Überraschenderweise blieben die in SCF plus Östradiol gezüchteten Klone viel länger unreif als die in SCF allein gezüchteten, wuchsen aber im Vergleich zu Klonen, gezüchtet in TGFα plus Östrogen oder SCF bzw. in TGFα plus Östrogen, viel langsamer. Da diese Klone keine Ähnlichkeit mit den typischen SCF/TGFα-Vorläufern hatten (hinsichtlich sowohl c-ErbB Expression und *in vitro* Lebensdauer, vgl. Beispiel 4), wurden sie nicht weiter untersucht.

### Beispiel 4

Untersuchung der *in vitro* Lebensdauer sowie der Expression von TGFαR/c-ErbB von SCF/TGFα-Vorläufern, die sich aus SCF-Vorläufern entwickelt hatten

Um zu untersuchen, ob die mittels LD-Klonierung von normalen Knochenmarkszellen oder SCF-Vorläufern in SCF, TGFα plus Östradiol mit großer Häufigkeit erhaltenen unreifen Klone tatsächlich typische SCF/TGFα-Vorläufer darstellten, wurden sie sowohl hinsichtlich ihrer *in vitro* Lebensdauer als auch ihrer Expression von TGFαR/c-ErbB und ihrer Proliferationsreaktion auf TGFα und andere Faktoren untersucht. Die Tests wurden sowohl im Vergleich mit den nur in TGFα plus Östradiol gezüchteten als auch mit Zellen aus SCF/TGFα-Vorläufer-Massenkulturen durchgeführt.

### a) Bestimmung der Lebensdauer

Für die Analyse der *in vitro* Lebensdauer wurden 10 bis 12 gesunde, unreife Kolonien, gezüchtet in SCF, TGFα und Östradiol (erhalten von 96-Well Platten mit 500 ausgesäten Zellen), oder gezüchtet in TGFα plus Östradiol (von Platten mit 15.000 Zellen), isoliert, suspendiert und in ihren jeweiligen Medien solange expandiert, bis in einer 100 mm Schale 20 x 10⁶ Zellen erhalten wurden oder die Zellen wegen Erreichen ihrer klonspezifischen in-vitro Lebensspanne (Zellaltern) das Wachstum einstellten. Die wachsenden Klone wurden dann passagiert (verdünnt und mit frischem Medium in neue Kulturschalen überführt), bis sie ebenfalls alterten. Alle unreifen Kolonien, die nach 13 Tagen Wachstum in SCF allein (6 Kolonien) erhalten wurden, sowie diejenigen aus den Kontrollkulturen (Östradiol allein: 5 Kolonien; SCF allein: 5 Kolonien; TGFα allein: 3 Kolonien; SCF plus TGFα: 8 Kolonien; SCF plus Östradiol: > 15 Kolonien) wurden ähnlich behandelt.

Klone, die die für die SCF/TGFα-Vorläufer vorausgesagte Lebensdauer zeigten, wurden nur in SCF, TGFα plus Östradiol erhalten, sowie, wie erwartet, in TGFα plus Östradiol. 8 von 12 der mit hoher Häufigkeit in SCF, TGFα plus Östradiol gewachsenen Klone hatten eine Lebensdauer von 23 bis über 28 Generationen (die verbleibenden 4 hatten eine Lebensdauer von 12 bis 15 Generationen). 7 von 10 Klonen, die in TGFα plus Östradiol mit niedriger Häufigkeit gewachsen waren, hatten eine ähnlich hohe Lebenserwartung (23 bis 31 Generationen; die Lebensdauer der verbleibenden 3 waren 15 bis 17 Verdoppelungen). Das zeigt deutlich, daß die Lebensdauer von SCF/TGFα-Vorläufern, die sich aus SCF-Vorläufern in Gegenwart von SCF, TGFα plus Östradiol entwickelt hatten, identisch derjenigen von echten SCF/TGFα-Vorläufern ist. Keine der Kolonien, die in Gegenwart einzelner Faktoren oder von SCF plus TGFα entstanden war, hatte eine Lebensdauer von mehr als 12 bis 16 Verdoppelungen. Ein Klon, erhalten in SCF plus Östradiol, konnte bis zur 22 Generation gezüchtet werden, während neun weitere eine kurze Lebensdauer hatten (12 bis 18 Verdoppelungen). Dieser Klon wuchs jedoch mit verminderter Geschwindigkeit, exprimierte sehr geringe Mengen von TGFαR/c-ErbB und reagierte in einem Wachstumsfaktor-Assay nicht auf TGFα. Es kann daher angenommen werden, daß diese Zellen eher ein anormaler Zellklon als tatsächliche SCF/TGFα-Vorläufer sind.

### b) Expression von TGFαR/c-ErbB und Reaktion auf TGFα und andere Wachstumsfaktoren

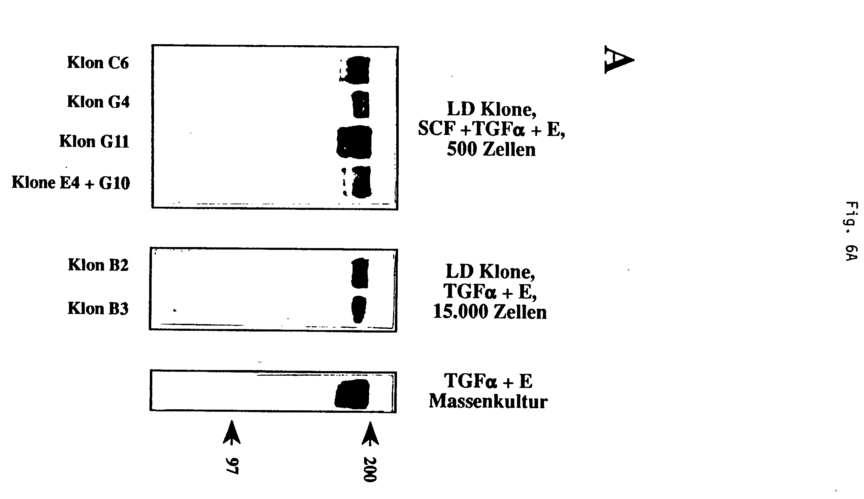

Um zu bestimmen, ob die mit großer Häufigkeit in SCF, TGFα plus Östradiol erhaltenen LD-Klone TGFαR/c-ErbB in ähnlichen Mengen exprimieren wie SCF/TGFα-Vorläufer, gezüchtet in TGFα plus Östradiol, wurden den Zellen von 5 LD-Klonen (2 Klone wurden aufgrund geringer Zellzahl zusammengefaßt) gezüchtet in allen drei Faktoren, von 2 Klonen, gezüchtet in TGFα plus Östradiol und von einer SCF/TGFα-Vorläufer-Massenkultur, jeweils alle Faktoren über Nacht entzogen, die Zellen lysiert und mittels Western Blot unter Verwendung von anti-c-ErbB-Antikörpern auf TGFαR/c-ErbB-Expression untersucht. Fig. 6A, Tafel A, (c-ErbB-Expression von LD-Klonen aus Knochenmark) zeigt deutlich, daß etwas schwankende, aber ähnliche Mengen von TGFαR/c-ErbB in allen drei Zelltypen exprimiert wurden, was wiederum nahelegt, daß die aus SCF-Vorläufern in Gegenwart aller drei Faktoren entstandenen Erythroblastenklone echte SCF/TGFα-Vorläufer sind.

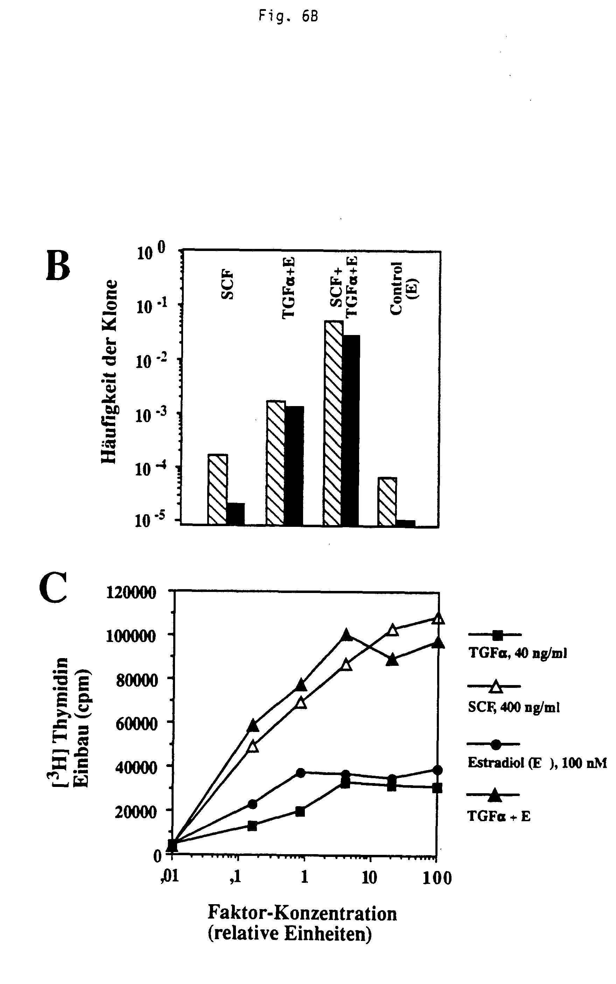

Um mehr quantitativ zu bestimmen, bis zu welchem Ausmaß die große Zahl von LD-Klonen, die in Gegenwart der drei Faktoren erhalten wird, SCF/TGFα-Vorläufern ähnlich ist, wurde ein anderer Weg gewählt: LD-Klone, induziert von gereinigten SCF-Vorläufern durch Kultivieren in den drei Faktoren (vgl. Fig. 5B) wurden am Tag 13 gezählt und diejenigen Platten ausgewählt, auf denen die Mehrzahl der Vertiefungen eine unreife Kultur enthielt. Dann wurde der Inhalt aller Vertiefungen suspendiert, in Medium ohne Faktoren gewaschen und in neue 96-Well Platten überführt, die Medium enthielten, das durch TGFα plus Östradiol ergänzt war. Kontroll-LD-Klone, erhalten aus TGFα plus Östradiol, SCF allein und Östradiol allein, wurden ähnlich behandelt. 3 Tage danach (Tag 16) wurden die Klone auf ihre Proliferationsfähigkeit untersucht, indem der [³H] Thymidineinbau gemessen wurde (Vertiefungen mit einer Zahl von Counts 5fach (bei den Einzelkolonien) oder 10fach (2 oder mehr Kolonien) über dem Untergrundwert wurden als positiv gezählt. Von dieser Analyse konnte die Häufigkeit der Thymidin einbauenden Klone berechnet werden (Fig. 6B, Tafel B; die gefüllten Balken zeigen die Thymidin-einbauenden Klone; die schraffierten Balken die Gesamtheit der Klone). Die erhaltenen Daten zeigen, daß im wesentlichen alle gesunden, unreifen Klone, die in TGFα plus Östradiol gezüchtet und am Tag 13 identifiziert worden waren, am Tag 16 Thymidin einbauten, was bestätigte, daß sie noch aktiv proliferierten. Dasselbe galt für mehr als 50 % der (30fach zahlreicheren) Klone, die in Gegenwart aller drei Faktoren entstanden waren. Im Gegensatz dazu bauten weniger als 10 % der wenigen Klone, die nach 13 Tagen in SCF allein überlebt hatten, Thymidin ein, während die ähnlich seltenen Klone, die in Gegenwart von Östradiol allein herausgewachsen waren, keinerlei Proliferation in TGFα plus Östradiol zeigten. Dies ließ vermuten, daß die in den Kontrollen entstandenen Klone keine typischen SCF/TGFα-Vorläufer darstellen, eine Feststellung, die durch ihre kurze *in vitro* Lebensdauer bestätigt wird (siehe a)).

Schließlich sollte bestätigt werden, daß die aus SCF-Vorläufern mit hoher Häufigkeit in Gegenwart aller drei Faktoren gewachsenen LD-Klone eine ähnliche Abhängigkeit von SCF, TGFα und Östradiol zeigen wie SCF/TGFα-Vorläufer. Fig. 6B, Tafel C zeigt, daß ein LD-Klon der Bezeichnung C6 (vgl. Fig. 6A, Tafel A) eine deutliche, konzentrationsabhängige Reaktion auf alle drei Faktoren zeigte, was beinahe dem Verhalten einer SCF-Vorläufer-Massenkultur, die 20 Tage lange in Gegenwart der drei Faktoren gezüchtet worden war (vgl. Fig. 3C), oder SCF/TGFα-Vorläufern, gezüchtet in TGFα plus Östradiol allein, entsprach.

### Beispiel 5

Definition der Faktoren, die für die Änderung des Differenzierungsprogramms von SCF-Vorläufern zu SCF/TGFα-Vorläufern erforderlich sind

Die in den vorangegangenen Beispielen erhaltenen Ergebnisse zeigen, daß sich SCF-Vorläufer zu SCF/TGFα-Vorläufern entwickeln können, d.h. daß sie sowohl die Fähigkeit für eine anhaltende Selbsterneuerung als auch zur Expression von endogenen TGFαR/c-ErbB erwerben, wenn sie in Gegenwart der drei Faktoren gezüchtet werden. Die Tatsache jedoch, daß solche Kulturen entscheidend abhängig von der Gegenwart von Hühnerserum sind, das geringe Konzentrationen von TGFα, SCF und/oder Östradiol sowie zusätzliche, nicht charakterisierte Faktoren enthalten kann, setzte der Auswertung der Daten Grenzen und warf mehrere Fragen auf. Es blieb unklar, ob SCF/TGFα-Vorläufer geringe Konzentrationen von SCF benötigten, welche allerdings im Hühnerserum vorhanden sind. Auch könnten SCF-Vorläufer geringe Mengen eines Hühnerfaktors benötigen, der TGFα funktionell ersetzt, und der ebenfalls im Hühnerserum enthalten ist. Zweitens war es unklar, zu welcher Zeit während der Entwicklung von SCF/TGFα-Vorläufern aus SCF-Vorläufern die verschiedenen Faktoren gebraucht wurden. Und schließlich blieb die Frage, welche(r) Faktor(en) im Hühnerserum für das TGFα/Östradiol-induzierte Auswachsen von SCF/TGFα-Vorläufern aus Knochenmark benötigt wird und ob diese(r) Faktor(en) eine neue Aktivität oder einen bekannten Faktor, z.B. SCF, darstellt bzw. darstellen, unbeantwortet.

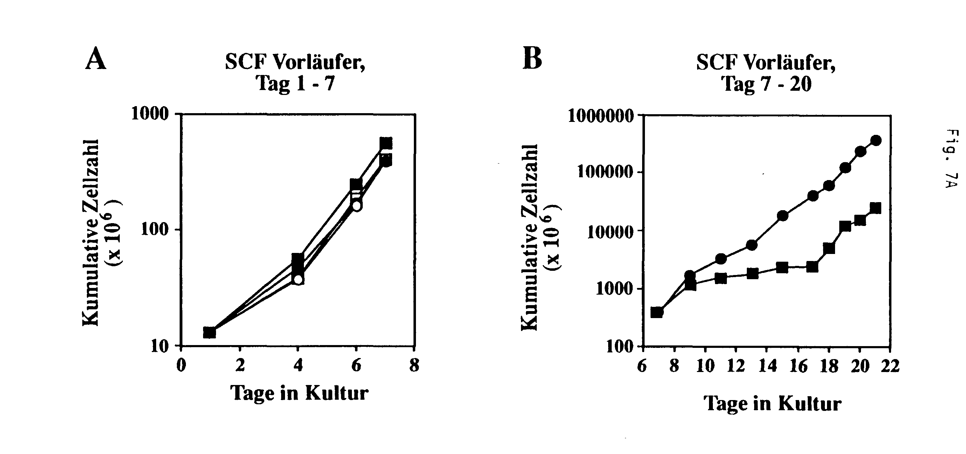

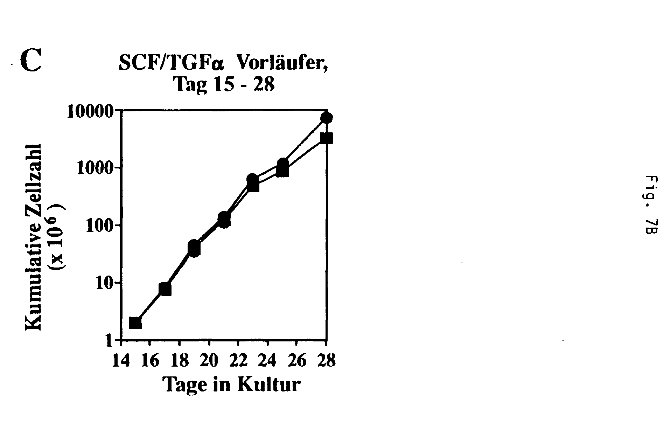

Um diese Fragen zu beantworten, war es notwendig, eine Charge von Hühnerserum herzustellen, die im wesentlichen frei war von endogenen Wachstumsfaktorund Hormonaktivitäten, die aber noch vollständig das Wachstum von faktorabhängigen Zellen ermöglichte, wenn die erforderlichen Wachstumsfaktoren von außen zugegeben wurden. Anfängliche Versuche hatten gezeigt, daß Tierkohle-behandeltes Hühnerserum (Schroeder et al., 1992) das TGFα/Östradiol-induzierte Auswachsen von SCF/TGFα-Vorläufern stark inhibierte (wenn auch nicht gänzlich unterband), jedoch die Wachstumsrate dieser Vorläufer nach ihrer Etablierung nicht beeinträchtigte. Es wurde daher für die vorliegenden Versuche Hühnerserum verwendet, welches mittels Freon-Behandlung und anschließender dreimaliger Tierkohle-Behandlung (Schroeder et al., 1992) gründlicher von endogenen Hormonen und Faktoren befreit worden war (im folgenden wird dieses depletierte Serum als "behandeltes Hühnerserum" bezeichnet). Knochenmarkszellen wurden in CFU-E-Medium, enthaltend Freon-behandeltes fötales Kälberserum und entweder unbehandeltes Hühnerserum oder behandeltes Hühnerserum. Die Zellen wurden entweder in SCF allein oder in SCF, TGFα und Östradiol gezüchtet und zu den in Fig. 7 angegebenen Zeitpunkten gezählt, wobei die kumulativen Zellzahlen, bestimmt wie in Beispiel 1, aufgetragen sind. CFU-E-Medium, welches mit dem behandelten Hühnerserum hergestellt wurde (in Fig. 7 bedeuten offene Quadrate gereinigtes Hühnerserum plus FCS; gefüllte Quadrate gereinigtes Hühnerserum plus Östradiol; offene Kreise normales Hühnerserum plus SCF und gefüllte Kreise normales Hühnerserum mit Östradiol), ermöglichte das Wachstum von SCF-Vorläufern in demselben Maß wie das Kontrollmedium mit unbehandeltem Hühnerserum, gleichgültig, ob die Zellen in SCF allein oder in SCF, TGFα plus Östradiol gezüchtet wurden (Fig. 7A, Tafel A). Es gab auch keine Auswirkung auf die Proliferationsrate von 15 Tage alten SCF/TGFα-Vorläufer-Kulturen, außer einen schwachen Effekt, wenn die Zellen zu altern begannen (Fig. 7B, Tafel C). Überraschenderweise verlangsamte jedoch das behandelte Hühnerserum die Entwicklung von SCF-Vorläufern zu SCF/TGFα-Vorläufern in Gegenwart von SCF, TGFα und Östradiol (Fig. 7A, Tafel B). Nach ihrem verspäteten Auftreten wuchsen jedoch die in behandeltem Hühnerserum entstandenen SCF/TGFα-Vorläufer mit derselben Geschwindigkeit wie die Kontrollzellen in unbehandeltem Hühnerserum, welche mindestens 5 Tage früher entstanden waren (Fig. 7A, Tafel B).

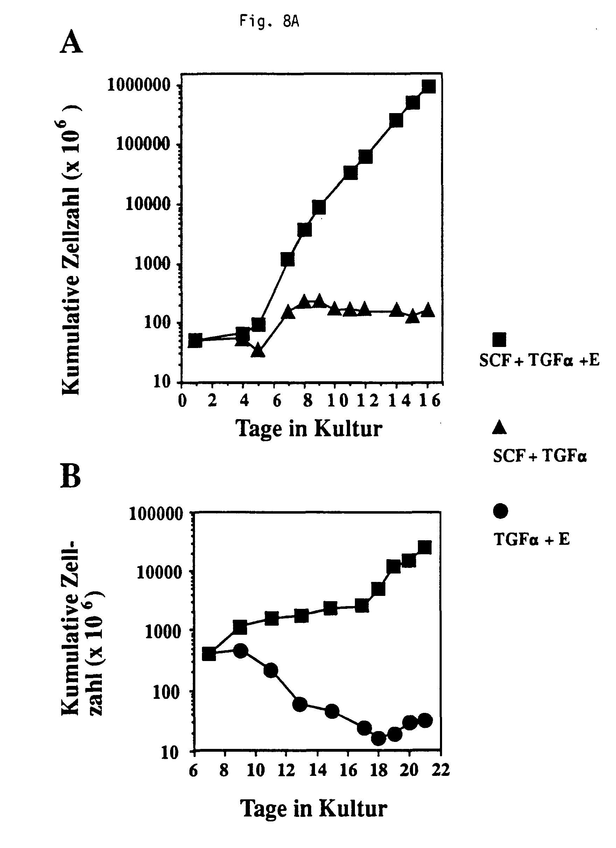

Diese Beobachtungen ließen verschiedene Schlußfolgerungen zu: Erstens enthält Hühnerserum eine zusätzliche Aktivität, die die Entwicklung von SCF-Vorläufern zu SCF/TGFα-Vorläufern fördert. Und zweitens ist diese Aktivität wichtig für die Umstellung in der Entwicklung, beeinträchtigt jedoch weder die Proliferation von SCF-Vorläufern vor der Änderung noch ist sie wichtig für die Proliferation von bereits etablierten SCF/TGFα-Vorläufern. Die Verfügbarkeit eines in geeigneter Weise behandelten Hühnerserums erlaubte auch die Untersuchung, zu welcher Zeit während der Entwicklung von SCF/TGFα-Vorläufern die bekannten Faktoren benötigt werden. 3 Tage alte gereinigte SCF-Vorläufer wuchsen mit vergleichbarer Geschwindigkeit in SCF plus TGFα, unabhängig von der Gegenwart oder dem Fehlen von Östradiol (Fig. 8A, Tafel A; das in dem verwendeten normalen Hühnerserum vorhandene Östradiol wurde wiederum mit ICI 164384 unterdrückt). Östradiol hat somit keine Auswirkung auf die frühe Proliferation von SCF-Vorläufern. Die Tatsache, daß sie mit gleicher Geschwindigkeit in Medien, enthaltend behandeltes Hühnerserum, SCF und Östradiol mit oder ohne TGFα wuchsen, zeigt, daß TGFα ebenfalls entbehrlich ist und daß der einzige von frühen SCF-Vorläufern benötigte Faktor SCF ist.

Ein unterschiedliches Muster der Anforderungen an Wachstumsfaktoren entsteht während der Änderung des Differenzierungsprogramms. Wie in Fig. 8A, Tafel A, gezeigt ist, hören in SCF plus TGFα ohne Östradiol gehaltene Zellen um den Tag 8 bis 10 irreversibel zu proliferieren auf, was nahelegt, daß Östradiol für das Umschalten erforderlich ist. Frühere Ergebnisse weisen darauf hin, daß es auch für die Proliferation von etablierten SCF/TGFα-Vorläufern erforderlich ist (Schroeder et al., 1993). Eine andere Gruppe von Versuchen zeigt deutlich, daß SCF während der Änderung des Differenzierungsprogramms erforderlich ist. 6 Tage alte SCF-Vorläufer, etabliert in Medien, enthaltend behandeltes Hühnerserum und SCF, können sich mit geringer Effizienz zu SCF/TGFα-Vorläufern entwickeln, wenn sie in behandeltem Hühnerserum, welches alle drei exogenen Faktoren enthält, weitergezüchtet werden. Wenn sie jedoch unter ansonsten identischen Bedingungen nur TGFα und Östradiol erhalten, fehlt ihnen diese Fähigkeit zur Gänze (Fig. 8A, Tafel B). Daher ist die Entwicklung von SCF/TGFα-Vorläufern von der Gegenwart von SCF während der Änderung des Differenzierungsprogramms abhängig, während dieselben Vorläufer, wenn sie einmal etabliert sind, von SCF unabhängig sind (s. Fig. 7B, Tafel C und unten). Schließlich brauchen SCF-Vorläufer kein TGFα (Fig. 7A, Tafel A), aber es findet in seiner Abwesenheit, sogar wenn unbehandeltes Hühnerserum verwendet wird, keine Bildung von SCF/TGFα-Vorläufern statt (Schroeder et al., 1993). Zusammenfassend erlauben die durchgeführten Versuche folgende Schlußfolgerung: Die gemeinsame Anwesenheit von SCF, TGFα und Östradiol ist für die Entwicklung von SCF/TGFα-Vorläufern aus SCF-Vorläufern erforderlich, während eine unbekannte weitere Aktivität in Hühnerserum die Effizienz ihrer Bildung erhöht.

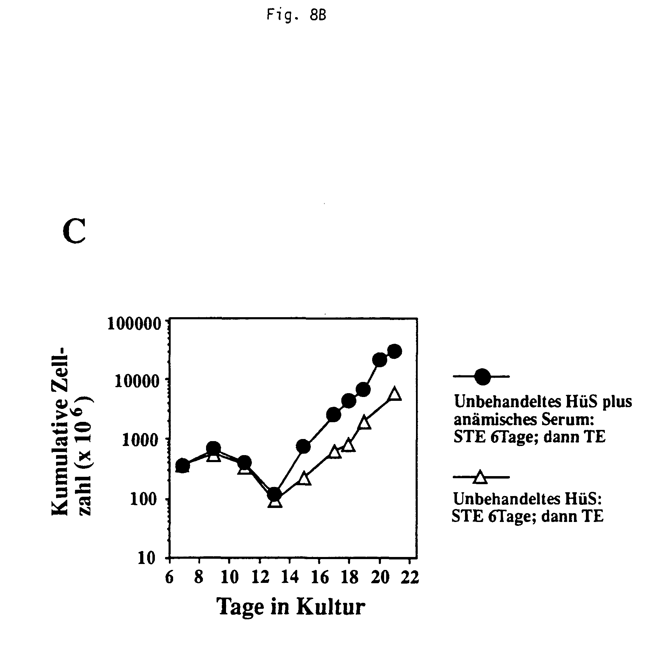

Einige Daten von orientierenden Versuchen, die ergänzend durchgeführt wurden, lassen vermuten, daß diese Aktivität Hühnererythropoetin sein könnte, beweisen dies aber nicht. Es wurde festgestellt, daß anämisches Serum in Wachstumsfaktor-Assays die Proliferation von SCF/TGFα-Vorläufern stark stimuliert; ein noch wichtigerer Befund war, daß anämisches Serum die Wachstumsrate von SCF/TGFα-Vorläufern während und nach deren Etablierung erhöhte, sogar wenn diese Zellen normalem Hühnerserum plus SCF, TGFα und Östradiol ("STE") ausgesetzt wurden (Fig. 8B, Tafel C).

Schließlich konnten Erythroblasten, die mittels eines Retrovirus stabil exprimiertes c-ErbB, also eine exogene Tyrosinkinase, zur Selbsterneuerung stimuliert worden waren und die nach Infektion mit einem weiteren Retrovirus den murinen Erythropoetin-Rezeptor exprimierten, durch humanes rekombinantes Erythropoetin (EPO) in ihrer Proliferationsrate dreifach und mehr stimuliert werden.

### Beispiel 6

Identifizierung zweier Faktoren aus Hühnerserum, die die Umwandlung von SCF-Vorläufern in SCF/TGFα-Vorläufer beschleunigen bzw. für deren Wachstum notwendig sind

### a) Liganden des Glucocorticoidrezeptors (z.B. Dexamethason) gehören zu den Faktoren aus Hühnerserum, die SCF Vorläufer für ihre Entwicklung zu SCF/TGPα-Vorläufern benötigen

Im vorigen Beispiel wurde gezeigt, daß die Entwicklung von SCF-Vorläufern zu SCF/TGFα-Vorläufern außer SCF, TGFα und Östradiol noch weitere, nicht definierte Faktoren aus Hühnerserum benötigt, die durch Aktivkohlebehandlung des Serums entfernt werden können. In Gegenwart eines aktivkohlebehandelten Hühnerserums findet die Entwicklung zu SCF/TGFα-Vorläufern nicht oder nur sehr ineffizient statt.

Da durch Aktivkohlebehandlung von Serum vor allem Steroidhormone entfernt werden, wurden weitere Steroidhormone neben Östradiol auf ihre Aktivität während der Umwandlung des Differenzierungsprogramms normaler erythroider Zellen untersucht. Es wurden zunächst Liganden des Glucocorticoidrezeptors getestet, da ein Mangel von Glucocorticoiden beim Menschen unter anderem zu einer Anämie führt und in Friend-Erythroleukämiezellen der Maus die DMSO-induzierte Differenzierung verhindert. Vorversuche ergaben 1) daß SCF-Zellen für ihre vorübergehende Selbsterneuerung kein DMSO brauchen und 2) daß etablierte SCF/TGFα-Zellen für ihr Wachstum geringe Konzentrationen an Glucocorticoiden brauchen. Die Zellen wachsen nicht, wenn sie in Gegenwart von TGFα und Östradiol in Medien kultiviert werden, in denen sowohl das fötale Kälberserum als auch das Hühnerserum aktivkohlebehandelt wurden. Wird zum gleichen Medium zusätzlich 1x10⁻⁶M Dexamethason zugegeben, werden die Zellen zum Wachstum mit normaler Geschwindigkeit stimuliert. Die Zellen können außerdem sogar in unbehandelten Medien dann nicht wachsen, wenn neben TGFα und Östradiol ein Glucocorticoid-Antagonist zugesetzt wird.

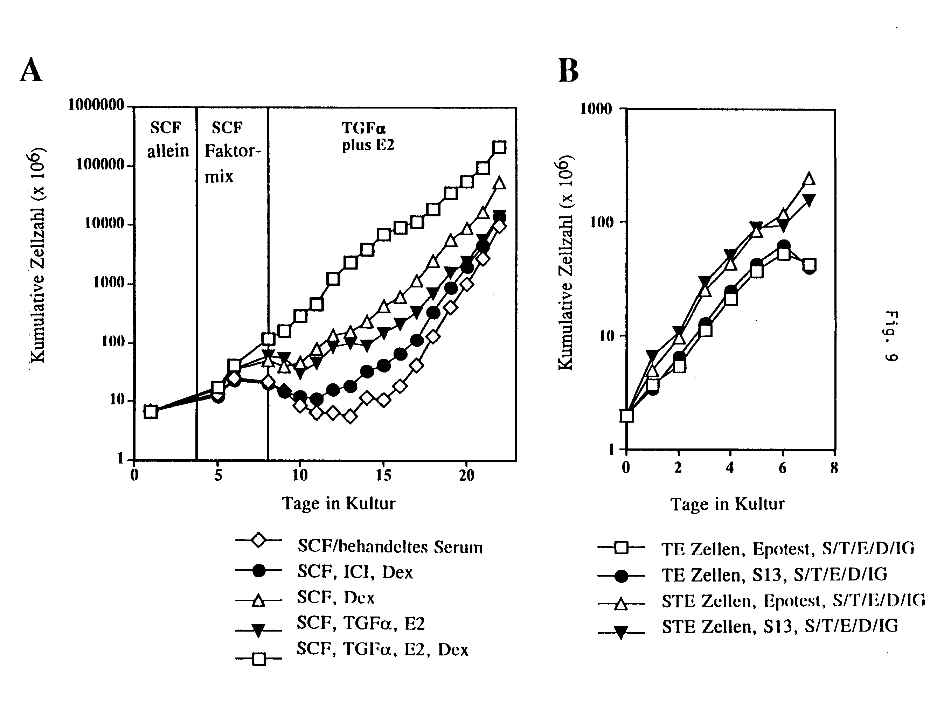

Um direkt zu testen, ob Glucocorticoide (Dexamethason) die Umwandlung von SCF- in SCF/TGFα-Vorläufer beschleunigen, wurde ein Experiment durchgeführt, in dem SCF-Vorläufer während einer kurzen Periode (4 Tage, Tag 3-7 nach Isolierung des Knochenmarks, im folgenden als Induktionsperiode bezeichnet) verschiedenen Faktor-Mischungen ausgesetzt wurden (siehe Fig. 9). Danach wurden die Zellen gewaschen und in Medium ausgesät, welches nur TGFα und Östradiol enthielt (TE Medium). In diesem Medium können nur c-ErbB exprimierende, fertig entwickelte SCF/TGFα Vorläufer, nicht aber SCF Vorläufer oder Zellen auf einem frühen Stadium der Entwicklung zu SCF/TGFα Vorläufern wachsen (siehe Beispiel 5). Die Ergebnisse sind in Fig. 9 dargestellt.

Als negative Kontrolle wurden die Zellen (4 Tage alte SCF-Vorläufer) während der Induktionsperiode in SCF plus Medium mit Aktivkohle-behandelten Seren (fötales Kälberserum und Hühnerserum) gezüchtet. Nach Umsetzen in TE Medium konnte über lange Zeit kein Zellwachstum beobachtet werden. Erst 9-10 Tage nach Umsetzen in TE-Medium wuchsen SCF/TGFα Vorläufer aus (Fig. 9, weiße Rauten), die sich wahrscheinlich von Zellen herleiteten, die bereits im Knochenmark als SCF/TGFα-Vorläufer vorlagen (siehe Beispiel 5).

Als positive Kontrolle wurden die SCF-Vorläufer während der 4 tägigen Induktionsperiode mit SCF, TGFα und Östradiol behandelt (Fig.9A, schwarze Dreiecke). Nach Umsetzen in TE Medium wuchsen, wie erwartet, sehr viel schneller Zellen aus, es wurde lediglich eine 5 tägige Verzögerung (lag-phase) des Auswachsens beobachtet. Dies entspricht den in Beispiel 1 (Fig. 1, Pfeile) dargestellten Ergebnissen.

Ein überraschendes Ergebnis wurde erhalten, wenn die Zellen während der Induktionsperiode mit SCF, TGFα Östradiol und Dexamethason behandelt wurden. Nicht nur wuchsen die Zellen während der Induktionsperiode viel schneller als in den Kontrollen, auch nach Umsetzen in TE Medium gab es keine feststellbare Verzögerung, die Zellen wuchsen mit gleichbleibender Geschwindigkeit weiter (Fig.9A, weiße Quadrate). Dieses Ergebnis zeigt, daß die Zugabe von Dexamethason die Umwandlung praktisch aller SCF-Vorläufer in SCF/TGFα Vorläufer bewirkte. Zusätzliche Untersuchungen mittels Phosphotyrosinblot (Western Blot mit Phosphotyrosin-Antikörpern) ergaben, daß diese Zellen die erwarteten Mengen an c-ErbB exprimierten.

Der Effekt von Dexamethason war auch an Zellen zu beobachten, die nur in Anwesenheit von SCF gezüchtet wurden. Zugabe von Dexamethason in Gegenwart von SCF beschleunigte das Auswachsen von SCF/TGFα Vorläufern stärker als in der positiven Kontrolle (SCF, TGFa, Östradiol, Fig. 9A, vgl. schwarze und weiße Dreiecke. Wie in Beispiel 5 zeigte sich, daß die Zellen außer SCF und Dexamethason das in den Seren in geringen Mengen enthaltene östradiol benötigten, da Zugabe des Östradiol-Antagonisten der Bezeichnung ICI 164384 (Schröder et al., 1993) das Auswachsen der Zellen auf das an der negativen Kontrolle beobachtete Maß begrenzte (Fig. 9A, vgl. weiße Rauten und schwarze Kreise). Außerdem benötigten die Zellen geringe Konzentrationen eines (unbekannten, im Hühnerserum enthaltenen) c-ErbB Liganden.

Diese Ergebnisse zeigen i) daß Dexamethason zusätzlich zu TGFα und Östradiol für das Wachstum von zur Selbsterneuerung fähigen SCF/TGFα Vorläufern notwendig ist und ii) daß dieses Hormon die Umwandlung von SCF-Vorläufern zu SCF/TGFα Vorläufern stark beschleunigt.

### b) Wachstum von SCF/TGFα Vorläufern; Insulin-like growth factor I (IGF-1) ersetzt zusammen mit SCF, TGFα, Östradiol und Dexamethason das für das Zellwachstum absolut erforderliche Hühnerserum.

Alle bisherigen Versuche mit normalen erythroiden, zur Selbsterneuerung fähigen Vorläuferzellen des Huhns waren an das Vorhandensein von getesteten Chargen von Hühnerserum gebunden; es konnten noch nicht alle Faktoren definiert werden, die Hühnerserum ersetzen können. Die in a) beschriebenen Ergebnisse, daß Dexamethason das Wachstum dieser Zellen in Aktivkohle-behandelten Hühnerseren ermöglicht, führten zu einer Reihe von neuen Versuchen, das Hühnerserum durch definierte Faktoren zu ersetzen. Die Definition dieser für Hühnerzellen erforderlichen Faktoren stellt die Grundlage für die eventuell entsprechenden Erfordernisse menschlicher Zellen dar.

Da eine Faktormischung von SCF, TGFα, Östradiol und Dexamethason sowohl die Entwicklung als auch das Wachstum von SCF/TGFα-Vorläufern optimal förderte, wurde diese Mischung in Medien mit und ohne Hühnerseren verwendet. Als mögliche weitere Faktoren zum Ersatz von Hühnerserum wurden Insulin-Like Growth Faktor (IGF-1) und aviäres IL-6 (Chicken Myelomonocytic Growth Factor, cMGF) untersucht. Die Versuche wurden in Medium mit (Fig. 9 B, S13 Medium) und ohne Hühnerserum (Fig. 9B, Epotest) durchgeführt.

Von den getesteten Faktoren war nur IGF-1 wirksam. Fig. 9B zeigt, daß in Gegenwart von SCF (S), TGFα (T), Östradiol (E), Dexamethason (D) und IGF-1 (IG) sowohl 16 Tage alte SCF/TGFα Vorläuferzellen (Fig. 9B, schwarze Kreise, weiße Dreiecke) als auch 9 Tage alte, in SCF/TGFα und Östradiol angezüchtete Knochenmarkszellen (Fig. 9B, schwarze und weiße Dreiecke) in Medien mit Hühnerserum (schwarze Symbole) und ohne Hühnerserum (weiße Symbole) gleich schnell proliferierten. Dieser Effekt war über einen Zeitraum von > 7 Tagen nachweisbar. In Abwesenheit von IGF-1 stellten die Zellen nach 2 Tagen ihr Wachstum komplett ein. Das gleiche Ergebnis (kein zellwachstum) wurde erhalten, wenn IGF-1 durch cMGF ersetzt wurde.

### Beispiel 7

Züchtung humaner erythroider Zellen, die Ähnlichkeit mit den Hühner-SCF- und Hühner-SCF/TGFα-Vorläufern haben

### a) Vorläufige Definition von Bedingungen, die das Auswachsen von humanen erythroiden Vorläufern aus Knochenmark oder peripherem Blut ermöglichen

Es wurden Versuche mit humanen hämatopoetischen Zellen durchgeführt. Die diesen Versuchen zugrundeliegende Annahme war, daß humane erythroide Vorläufer eine ähnlich lange *in vitro* Lebensdauer wie humane Fibroblasten (50 bis 70 Generationen) haben, was die Grundlage für den Nachweis von humanen selbsterneuerungsfähigen erythroiden Vorläufern darstellt.

Als Quelle für diese Versuche diente entweder Knochenmark oder peripheres Blut von gesunden Spendern. Aus diesen Quellen wurden unreife Blutzellen, die das CD34-Zelloberflächenantigen exprimieren, mittels Immunaffinitätschromatographie nach der von Shpall et al., 1994, beschriebenen Methode angereichert. Die angereicherten Zellen wurden, wie in den vorigen Beispielen, in ein modifiziertes CFU-E-Medium (Hayman et al., 1993), enthaltend humanes Serum (Sigma) statt Hühnerserum und eisengesättigtes humanes Transferrin (Sigma) statt Conalbumin, ausgesät. Das Medium wurde ergänzt mit 20 ng TGFα (Promega), 20 ng rekombinantem EGF (Promega; EGF wurde verwendet für den Fall, daß das auf erythroiden Zellen vorhandene hypothetische Mitglied der EGF-Rezeptorfamilie nicht TGFα als funktionellen Liganden hat), 100 ng gereinigter humaner SCF (Promega), 5 x 10⁻⁷ M Östradiol (in einigen Versuchen, die der Charakterisierung der Zellen dienten, wurden dem Medium noch weitere Faktoren wie IL-3, IL-1 und LIF zugesetzt). Das Zellwachstum wurde mittels Zellzählung verfolgt und die in den Kulturen vorhandenen Zelltypen wurden mittels Zytozentrifugation auf Objektträger und histochemische Färbung auf Hämoglobin und histologischen Farbstoffe analysiert (Beug et al., 1982).

### i) Versuche mit Knochenmark

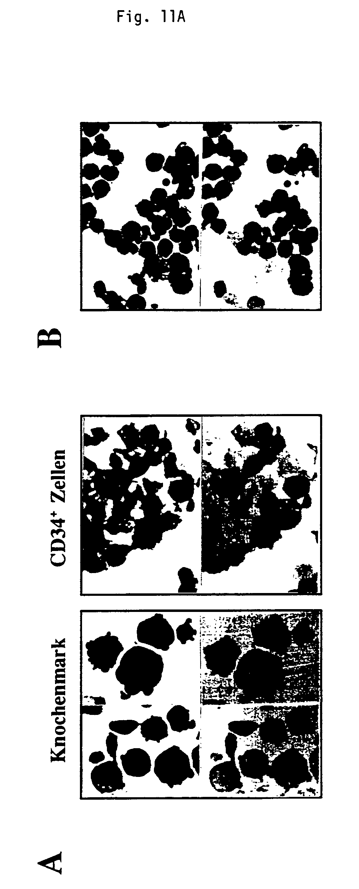

Die anfänglichen Versuche, erythroide Vorläufer aus menschlichem Knochenmark in modifiziertem CFU-E-Medium, enthaltend Humanserum, eisengesättigtes humanes Transferrin, 20 ng TGFα (Promega), 20 ng rekombinanten EGF (Promega), 100 ng gereinigten humanen SCF (Promega), 5 x 10⁻⁷ M Östradiol und verschiedene andere Faktoren (je 10 ng IL-3, IL-6, IL-1 und LIF) pro Milliliter (ml) zu züchten, zeigten zunächst keinen Erfolg. Wenn jedoch zum Medium rekombinantes EPO (3 Internationale Einheiten/ml) zugegeben wurde, konnten erythroide Vorläufer herausgezüchtet werden, die 13 Tage lang unreif blieben, aber im wesentlichen alle am Tag 16 differenziert waren. Während dieser Zeit nahmen die Zellzahlen um das 25 bis 50fache zu; eine genauere Bestimmung war aufgrund niedriger Zellzahlen (nur 2 x 10⁶ anfänglich ausgesäte Zellen, deshalb nach 3-5 Tagen weniger als 10⁵ Zellen) nicht möglich. Die erhaltenen proliferierenden Zellen ähnelten humanen Proerythroblasten, überraschenderweise waren sie den normalen erythroiden Hühner-Vorläuferzellen ähnlich (Fig. 11A, Tafel A und B, siehe unten). Während der ersten paar Tage der Kultur, ebenso wie nach Tag 15, waren viele, kernhältige Reticulozyten, den Kern ausstoßende Zellen und Erythrozyten sichtbar, was darauf hindeutet, daß die differenzierenden Reticulozyten in den Kulturen normal zu Erythrozyten differenzierten und auch den Vorgang der Enukleation (Kernausstoßung) normal durchführen konnten. Ohne EPO wuchsen die Kulturen nicht und enthielten sehr wenige unreife erythroide Zellen. Sie enthielten hauptsächlich heranreifende Monoblasten sowie verschiedene Arten von unreifen Granulozyten (Neutrophile, Eosinophile, Mastzellen).

### ii) Versuche mit Zellen aus peripherem Blut

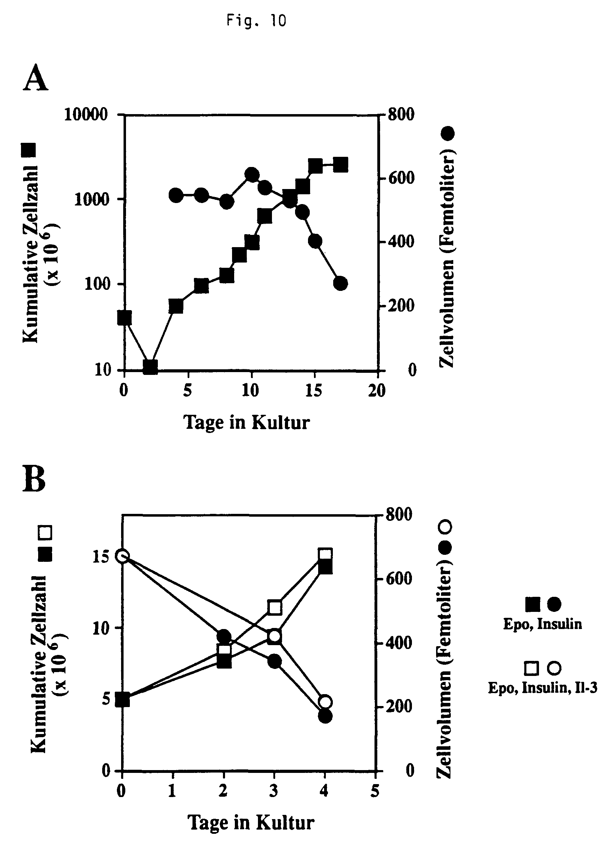

Das in i) beschriebene Experiment wurde mit 40 x 10⁶ CD34⁺-Zellen, angereichert aus humanen peripherem Blut, wiederholt. 2 x 10⁶ Zellen/ml in modifiziertem CFU-E-Medium plus SCF, TGFα und EGF, Öatradiol und humanes rekombinantes EPO wurden in Gewebekulturschalen ausgesät und die Zellen zu den angegebenen Zeiten gezählt, wobei die durchschnittlichen Zellvolumina in einem elektronischen Zellzähler des Typs CASY-1, Schärfe System, bestimmt wurde. Da die Ausgangszellzahl größer war als bei dem mit Knochenmark durchgeführten Versuch, konnte die Proliferationskinetik der Kultur genau verfolgt werden. Fig. 10A zeigt, daß die Zellzahlen während der ersten 2 bis 3 Tage abnahmen, was auf die Reifung und/oder den Zelltod von teilweise differenzierten Vorläufern zurückzuführen ist. Anschließend proliferierten die Zellen exponentiell mit Verdopplungszeiten zwischen 20 und 30 h bis zum Tag 15, nach welchem kein weiteres Wachstum beobachtet wurde. Die Gesamtzunahme der Zellzahl während dieser Wachstumsphase war > 300fach. Fig. 10A zeigt auch, daß die Zellen während der Phase des exponentiellen Wachstums ihre Größe behielten (Zelldurchmesser zwischen 9 und 10 µm, Zellvolumina zwischen 500 und 600 Femtoliter), was ein erster Hinweis darauf ist, daß sie unreif blieben.

Da antigens Marker, die humane Proerythroblasten *von* anderen myeloiden oder multipotenten Vorläufern unterscheiden, für die Durchführung dieser Versuche nicht zur Verfügung standen, und der Nachweis durch histologische Färbung nicht wirklich definitiv ist, wurden indirekte Methoden eingesetzt, um den Prozentsatz von erythroiden Vorläufern in den Kulturen zu bestimmen: Erstens wurden Aliquots in regelmäßigen Zeitabständen mittels saurem Benzidin gefärbt, was einen sehr empfindlichen Hämoglobinnachweis darstellt (Graf und Beug, 1978). Am 6. Tag enthielten die Kulturen bereits 14 % Benzidin-positive Zellen, an den Tagen 10 und 11 waren diese Werte auf 51 bzw. 63 % angestiegen. Da eine reine Kultur von Hühner-SCF/TGFα-Vorläufern zwischen 30 und 60 % Benzidin-positive Zellen enthält, läßt sich aus diesen Ergebnissen schließen, daß ca. am Tag 10 die Kultur vorwiegend aus erythroiden Vorläufern bestand. Diese Ansicht konnte in einem Versuch bestätigt werden, in dem die Zellen induziert wurden zu differenzieren: Ein Aliquot der 10 Tage alten Kultur wurde gewaschen und in modifiziertem CFU-E-Medium, enthaltend 10 E/ml humanes rekombinantes EPO plus 10 ng/ml Insulin bzw. IGF-1 (Insulin like growth factor 1) resuspendiert. Ein Parallel-Aliquot erhielt zusätzlich IL-3 (10 ng/ml). Die Daten in Fig. 10B zeigen, daß die Zellzahlen ungefähr um das 3fache zunahmen, wobei ihr Zellvolumen gleichzeitig beträchtlich abnahm, wie es für differenzierende erythroide Zellen zu erwarten ist. Eine nach 2 Tagen vorgenommene saure Benzidinfärbung ergab > 95 % Benzidin-positive Zellen in der Kultur, die EPO/Insulin allein erhalten hatte. Dies zeigt an, daß die Mehrzahl der vor der Induktion der Differenzierung vorhandenen Zellen erythroid gewesen sein muß, insbesondere weil in den differenzierenden Kulturen nach Zytozentrifugation und histologischer Färbung (siehe unten) sehr wenige apoptotische Zellen sichtbar waren. Der Zusatz von IL-3 verzögerte wahrscheinlich die Differenzierung; nach 2 Tagen wurden nur 66 % Benzidin-positive Zellen nachgewiesen, und die Zellen wuchsen etwas schneller, während ihr Zellvolumen langsamer abnahm (Fig. 10B).

### b) Charakterisierung der in SCF, TGFα, Östradiol und EPO proliferierenden Zellen

Um zu bestimmen, ob die in a) durch Kultur in SCF, TGFα plus EGF, Östradiol und rekombinantem EPO erhaltenen erythroiden Vorläufer den in den vorangegangenen Beispielen gezüchteten Hühner-SCF/TGFα-Vorläufern entsprechen, wurden die folgenden zwei Versuchsansätze gewählt:

Erstens wurden die in den Kulturen vorhandenen Zelltypen mittels Zentrifugation auf Objektträger und kombinierte histologische und histochemische Färbung auf Hämoglobin charakterisiert (Beug et al., 1992, siehe die dort definierten Stadien). Damit sollte bestimmt werden, wie lange sich unreife, Hämoglobinnegative oder schwach positive Proerythroblasten in den Kulturen halten würden, um einen Hinweis dafür zu erhalten, ob die erythroiden Vorläufer tatsächlich eine Selbsterneuerung durchmachten, wie aufgrund von Wachstumskinetik und Größenverteilung (Fig. 10A) zu vermuten war. Proerythroblasten unterscheiden sich in der angewandten Färbung von anderen Zellen durch einen zentralen, großen Zellkern, stark basophiles Zytoplasma, charakteristische Lappung des Zytoplasmasaums und eine schwache, jedoch von myeloiden Zellen unterscheidbare Anfärbung mit neutralem Benzidin. Fig. 11A, Tafel A (proliferierende Zellen, Knochenmark nach 7 Tagen, CD34⁺-Zellen nach 10 Tagen) und B (differenzierte Zellen nach 10 Tagen Proliferation und 4 Tagen Differenzierung), zeigt, daß ein großer Prozentsatz der Zellen, die sich in der Kultur halten, Benzidin-negativen Protoerythroblasten ähneln, daneben gab es einige myeloide Zellen. Diese Ergebnisse wurden bis zum Tag 14 erhalten, danach nahm der Prozentsatz an reifenden Zellen deutlich zu. Im Gegensatz dazu stellten die nach 4 Tagen Differenzierungsinduktion erhaltenen Zellen (siehe oben) Reticulozyten sowie den Kern ausstoßende und reife Erythrozyten dar (Fig. 11A, Tafel A und B), was eine weitere Bestätigung dafür ist, daß die in SCF, TGFα, Östradiol und EPO gehaltenen Zellen tatsächlich daran gehindert waren, in die durch die obigen Faktoren induzierte Differenzierung einzutreten. Fig. 11A zeigt durch Zentrifugation auf Objektträger und kombinierte histologische und histochemische Färbung auf Hämoglobin charakterisierte Präparationen von humanem Knochenmark (BM) und CD34⁺-Zellen (CD34) (Tafel A), jeweils photographiert unter grünem Licht (oben) und blauem Licht (unten), um histologische Einzelheiten und Hämoglobinfärbung festzustellen. Er = Erythrozyten und kernausstoßende Erythrozyten; R = Reticulozyten; Pe = Proerythroblasten; M = myeloide Zellen. Fig. 11A, Tafel B zeigt 10 Tage lang kultivierte CD34⁺-Zellen, die 4 Tage lang zur Differenzierung induziert und auf ähnliche Weise photographiert wurden.

Ein deutlicherer Beweis, daß aus humanen erythroiden Vorläufern tatsächlich Zellen ähnlich SCF/TGFα-Vorläufern erhalten werden können, wurde erhalten, indem untersucht wurde, ob die Zellen sowohl c-Kit als auch ein Mitglied der c-ErbB/EGF-Rezeptorfamilie exprimieren und als Reaktion auf die jeweiligen Liganden proliferieren. Da zunächst keine Kulturen erhalten werden konnten, die während der erwarteten 50 bis 70 Teilungen eine Selbsterneuerung durchmachten, wurde in Betracht gezogen, daß die Mehrzahl der Zellen in den Kulturen, besonders in frühen Stadien, SCF-Vorläufern entsprechen und daß SCF/TGFα-Vorläufer nur mit geringer Effizienz entstanden waren, was wahrscheinlich auf suboptimale Kulturbedingungen zurückzuführen ist. Es wurde daher die Reaktivität der humanen Knochenmarkszellen auf verschiedene Wachstumsfaktoren mittels verschiedener Wachtstumsfaktor-Assays getestet (Leutz et al., 1984; Hayman et al., 1993). Die Ergebnisse dieser Assays sind in Fig. 11B, Tafel C (Selbsterneuerungsfaktoren TGFα/EGF, SCF) und Fig. 11B, Tafel D (Differenzierungsfaktoren (EPO, IL-3)) dargestellt. Für diese Versuche wurden CD34⁺-Zellen 8 Tage lang gezüchtet, gewaschen und, wie in Beispiel 4 beschrieben, auf ihre Wachstumsfaktorabhängigkeit getestet mit dem Unterschied, daß CFU-E-Medium ohne Humanserum verwendet wurde. Eine relative Wachstumsfaktorkonzentration von 100 entsprach 400 ng/ml rekombinantem SCF, je 40 ng/ml TGFα bzw. EGF; 10 ng/ml humanes rekombinantes IL-1, 20 E/ml humanem rekombinantem EPO, 40 ng/ml humanem rekombinantem IL-3 und 10 ng/ml rekombinantes Maus-LIF. Die dargestellten Werte sind die Durchschnittswerte von Dreifachbestimmungen. Die Zellen zeigten eine starke Reaktion auf SCF und, was noch bedeutsamer war, eine schwache, aber eindeutige Reaktion auf eine Mischung von TGFα und EGF. Hingegen wurde keine Reaktion auf die beiden Zytokine IL-1 und LIF, die auf sehr frühe, multipotente hämatopoetische Zellen wirken, beobachtet. Dies läßt darauf schließen, daß die auf SCF reagierenden Zellen "committed" erythroide Vorläufer darstellen. Wie erwartet, reagierten die Zellen ebenfalls stark auf die erythroiden Differenzierungsfaktoren EPO und IL-3, was ebenfalls bestätigt, daß die Kultur vorwiegend erythroide Zellen enthält.

### Beispiel 8

Zusätzliche Wachstumsfaktoren und Steroidhormone induzieren in Kulturen von zur terminalen Differenzierung fähigen, menschlichen Proerythroblasten die Selbsterneuerung für lange Zeit (> 20 Generationen).

In Beispiel 6 wurden im Hühnersystem Ergebnisse erhalten, die von potentieller Bedeutung für das Auswachsen von menschlichen, zur Selbsterneuerung fähigen Proerythroblasten waren:
1. Von den zunächst nicht definierten Faktoren im Hühnersystem, die zusätzlich zu SCF, TGFα und Östradiol für die Entwicklung von SCF Vorläufern zu SCF/TGFα Vorläufern notwendig sind, konnten zwei Faktoren identifiziert werden: das Steroidhormon Dexamethason und der generelle Wachstumsfaktor Insulin-like Growth Faktor (IGF)-1.
2. Es wurde daher die Wirkung von Dexamethason auf das Selbsterneuerungsverhalten menschlicher Proerythroblasten genauer untersucht. Ebenso wurde untersucht, ob IGF-1, welches im Hühnersystem die Zellen von Hühnerserum unabhängig macht, auf menschlichen Zellen zumindestens wachstumsfördernde Eigenschaften zeigt. Die erhaltenen Ergebnisse waren überraschend: es gelang, die Vermehrung menschlicher Proerythroblasten während ihrer Lebensdauer *in vitro* von 200 - 1.000 fach auf über 100.000 fach zu steigern. Dies ermöglichte eine genauere Charakterisierung der erhaltenen Zellpopulationen durch Kolonie-Tests und FACS- Analyse. Auch konnte das Differenzierungsverhalten der Proerythroblasten viel genauer als in Beispiel 7 untersucht werden, weil genügend Zellen zur Verfügung standen.

### a) Wirkung von Dexamethason

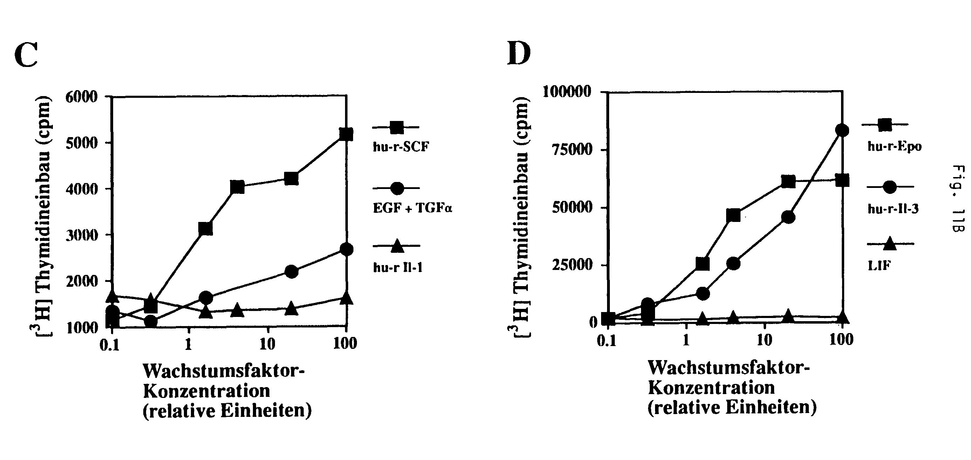

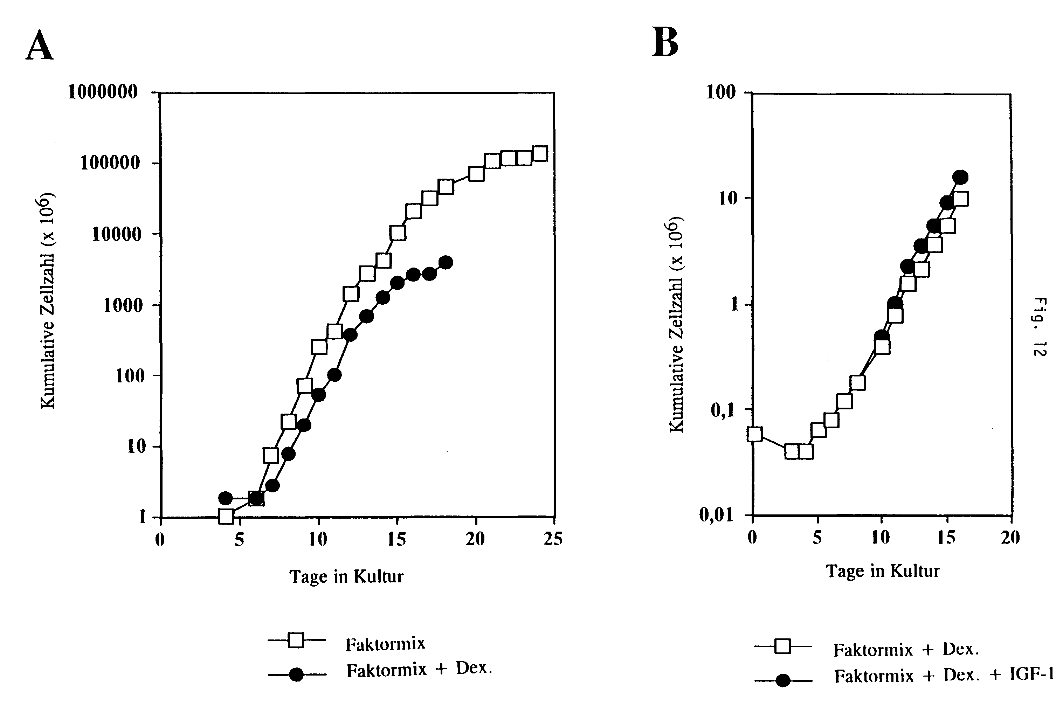

Menschliche, CD34⁺ Zellen aus Nabelschnurblut, gereinigt wie in Beispiel 7, Teil (ii) beschrieben, wurden in Medium plus Epo, huSCF, TGFα, und Östradiol ausgesät, wie in Beispiel 7 beschrieben. Zu einer zweiten Kultur wurden zusätzlich zu obigen, als "factor mix" bezeichneten Faktoren 1x10⁶ M Dexamethason zugegeben. Das Zellwachstum wurde bis zum Aufhören erkennbarer Vermehrung verfolgt. Die Ergebnisse sind in Fig. 12A dargestellt. Wie erwartet, wuchsen Zellen in "factor mix" bis Tag 13/14 exponentiell und zeigten eine 1.000 - 2.000 fache Zellvermehrung (Fig. 12A, schwarze Kreise). Unerwarteterweise proliferierte die Parallelkultur, die in "factor mix" plus Dexamethason gezüchtet wurde, bis mindestens Tag 18 exponentiell und stellte auch danach nur allmählich ihr Wachstum ein (Fig. 12A, weiße Quadrate), so daß eine 150.000 fache Zellvermehrung erhalten wurde. Geht man davon aus, daß immer ein Teil der Zellen in die spontane Differenzierung eintritt und damit nur ein Teil der unreifen Zellpopulation für die Aufrechterhaltung des Selbsterneuerungs-Potentials der Kultur zur Verfügung steht, so zeigen diese Daten, daß die Proerythroblasten aus menschlichem Nabelschnurblut in Gegenwart von Epo, SCF, TGFa, Östrogen und Dexamethason in der Lage sind, ihr Selbsterneuerungspotential für mindestens 20 Zellgenerationen aufrechtzuerhalten. Dies ist wesentlich mehr als selbst die 7-10 Zellteilungen, die menschliche BFU-Es innerhalb ihres normalen Entwicklungspotentials (Sawada et al., 1990) durchlaufen. Somit ist gezeigt, daß menschliche erythroide Vorläuferzellen, ähnlich wie die entsprechenden Zellen des Huhns, durch Kombinationen von Tyrosinkinase-Liganden und Liganden von Steroidrezeptoren zu einer echten Änderung ihres Entwicklungspotentials, d.h. zu langandauernder Selbsterneuerung, induziert werden können.

### b) Wirkung von IGF-1

CD34+ Zellen aus peripherem Blut eines Erwachsenen (gewonnen wie in Beispiel 7, Teil ii)) wurden in "factor mix" plus Dexamethason kultiviert, bis die Zellen exponentiell zu wachsen begannen. Dann wurde einem Aliquot der Zellen zusätzlich 40 ng/ml humaner, rekombinanter IGF-1 (Promega) zugesetzt. Wie Fig. 12B zeigt, wuchsen die Zellen mit IGF-1 (schwarze Kreise) deutlich schneller als ohne diesen Wachstumsfaktor (weiße Quadrate). Da das verwendete Medium 15 % fötales Kälberserum sowie 4 % Serum von menschlichem Nabelschnurblut enthielt (von denen zu erwarten ist, daß sie eine basale Konzentration von IGF-1 enthalten), wurde eine zwar relativ geringe Steigerung der Wachstumsrate erhalten; die jedoch zeigt, daß die Zellen auf IGF-1 reagieren. Dies geht auch aus Versuchen hervor, bei denen die Zellen über Nacht ohne Faktor kultiviert wurden, dann 5 und 10 Minuten mit IGF-1 stimuliert, lysiert und im Phosphotyrosin-Blot (siehe Beispiel 2) auf Rezeptorphosphorylierung untersucht wurden. Die so behandelten Zellen zeigten im Phosphotyrosinblot Autophosphorylierung der intrazellulären 90 kD IGF-1-Rezeptor-Kette sowie des 130 kD IRS-1 ("insulin receptor Substrate") Proteins.

### c) Genauere Charakterisierung der zur Selbsterneuerung fähigen menschlichen Erythroblasten mit Hilfe von Kolonietests und Oberflächenmarkern

Die überraschende Fähigkeit der in "factor mix" mit und ohne Dexamethason gezüchteten, menschlichen Proerythroblasten ließ es sinnvoll erscheinen, diese Zellen genauer auf ihr Entwicklungspotential und ihre Stellung innerhalb der erythroiden Entwicklungsreihe zu untersuchen. Hierfür wurden zwei Arten von Methoden angewendet:

Erstens wurden die Zellen in halbflüssigen Medien mit geeigneten Kombinationen von Zytokinen ausgesät und 10 Tage später die Art der gewachsenen Kolonien ausgezählt. Folgende Kolonietypen wurden unterschieden: i) burst forming unit erythroids (BFU-E), aus 1.000 bis >20.000 Zellen bestehende Kolonien, die ausschließlich Erythrozyten enthalten und somit zeigen, daß die Ausgangszelle ein unreifer Vorläufer ist, der auf die erythroide Entwicklungsreihe festgelegt ("committed") ist; ii) BFU-mix, große Kolonien mit > 20.000 Zellen, die außer Erythrozyten Zellen mindestens einer anderen Entwicklungsreihe enthalten und somit auf multipotente Ausgangszellen hindeuten; und iii) colony-forming units granulocyte/macrophage (CFU-GM), Kolonien aus 100 bis >1.000, Zellen, die keine Erythrozyten, sondern nur myeloide Zellen (Makrophagen und/oder Granulozyten) enthalten und somit von nicht-erythroiden Vorläuferzellen abstammen.

Zweitens wurden die Zellen mit Hilfe geeigneter Antikörper und FACS-Analyse auf die Expression von Oberflächenmarkern getestet, die für Zellen bestimmter Entwicklungsreihen und Reifegraden spezifisch sind. Obwohl keine Oberflächenmarker existieren, die einzeln verwendet, ausschließlich menschliche Proerythroblasten erkennen, kann durch Kombination mehrerer Marker, die auf unreifen und/oder reifen Erythroblasten exprimiert sind, mit spezifischen Markern für myeloide Zellen (CD 33) und lymphoide Zellen (CD-3, CD-19) die Zugehörigkeit der Zellen zur erythroiden Entwicklungsreihe mit großer Sicherheit bestimmt werden. Hierfür eignet sich vor allem der CD 71 (α-Transferrin Rezeptor) Antikörper, der zwar alle proliferierenden Zellen schwach anfärbt, erythroide Zellen jedoch sehr stark markiert. Zusammen mit CD 117 (c-Kit, nur auf ganz unreifen, BFU-E ähnlichen, erythroiden Zellen sowie multipotenten Vorläufern und bestimmten myeloiden Zellen (Mastzellen) exprimiert) und GPA (α-Glykophorin, spezifisch für teilweise reife erythroide Zellen) erlaubt der CD-71 Antikörper die sichere Bestimmung von Proerythroblasten (CD 71 bright, CD 107 positiv bis schwach positiv, GPA negativ oder schwach positiv, CD33, CD3, CD 19 negativ).

Um Zellen aus den entsprechenden Kulturen auf ihre Entwicklungsreihen-Zugehörigkeit und ihren Reifegrad innerhalb der erythroiden Entwicklungsreihe zu testen, wurden Zell-Aliquots aus den in Fig. 12A gezeigten Kulturen am Tag 13 und Tag 16 entnommen und den in den Tabellen I bis III dargestellten Tests unterworfen. Die am Tag 16 entnommenen Zellen aus der Kultur ohne Dexamethason wurden außerdem nach Dichte aufgetrennt: im Hühnersystem sind nur Zellen mit einer Dichte < 1.070 g/cm³ unreif, Zellen mit einer Dichte > 1.072 g/cm³ sind in allen Fällen teilweise reif und benötigen nur noch 1-2 Tage bis zur Reifung zu Erythrozyten, wobei sie nur wenige Zellteilungen durchlaufen. Entsprechende Fraktionen wurden von den menschlichen Zellen präpariert und getrennt auf Koloniebildung und Oberflächenmarker getestet.
Die Ergebnisse sind in Tabellen I bis III dargestellt. Sie zeigen, daß der überwiegende Kolonietyp sowohl nach 13 wie auch nach 16 Tagen von BFU-Es, also von unreifen erythroiden Vorläufern gebildet wird. Der Effekt von Dexamethason, die Zellen länger in einem unreifen, durch Selbsterneuerung charakterisierten Zustand zu halten, wird auch durch die BFU-E Zahlen deutlich: die Kulturen mit Dexamethason enthalten sowohl nach 13 wie auch nach 16 Tagen die 2-2.5 fache Zahl an unreifen, koloniebildenden erythroiden Vorläufern wie ohne Dexamethason. Die Daten zeigen auch, daß die Zellpopulation zum ganz überwiegenden Teil aus "committed" erythroiden Vorläufern bestehen. Sowohl nach 13 wie nach 16 Tagen sind mehr als 90 % der koloniebildenden Zellen rein erythroide Vorläufer, der Anteil der multipotenten (BFU-mix) und myeloid "committed" Vorläufer beträgt jeweils nur 3-6 %. Interessanterweise stimuliert Dexamethason auch den Gehalt an multipotenten Vorläufern 3-6 fach, während der Effekt auf die myeloiden Vorläufer viel schwächer ausfällt.

Wie erwartet, war die dichtere Fraktion (> 1.072 g/cm³) aus 16 Tage alten Nabelschnurblut-Zellen unfähig, in halbflüssigen Methocel-Medium Kolonien zu bilden. Ihr reiferer Charakter wurde auch durch die Marker-Analyse bestätigt.

Die Tabellen II und III zeigen weiterhin, daß die Ergebnisse der Marker-Analyse im FACS die Schlußfolgerungen aus den Kolonietests voll bestätigen. Alle Kulturen enthielten nur einen geringen Anteil an myeloiden Zellen (10-20 % nach 13 Tagen, 5-7 % nach 16 Tagen, keine lymphoiden Zellen sowie kaum CD34 positive Zellen (um 5 % Daten nicht gezeigt. Um 85 % der Zellen sind stark CD71 positiv, jedoch nur wenige % der Zellen sind GPA positiv. Der Effekt von Dexamethason, die Vorläuferzellen in einem unreifen Zustand zu halten, wird auch durch die CD 107 (c-Kit) Expression deutlich: am Tag 16 (wo die Kultur ohne Dexamethason schon deutlich in der Wachstumsgeschwindigkeit abnahm; siehe Fig. 12A), sind nur noch 21% der Zellen c-Kit positiv, während in der in Gegenwart von Dexamethason gehaltenen, noch exponentiell wachsenden Parallelkultur noch über 50 % der Zellen c-Kit positiv waren. Auch der partiell reife Zustand der dichteren Fraktion aus der 16 Tage alten Kultur konnte durch Markeranalyse bestätigt werden: nur noch 53 % der Zellen waren CD71 positiv (reifende Zellen verlieren den Transferrinrezeptor) während 66 % der Zellen GPA positiv waren.

Zusammengefaßt ergab die Charakterisierung der Kulturen aus Nabelschnurblut folgendes:
i) Die Kulturen bestehen ganz überwiegend aus unreifen, proerythroblastenähnlichen Vorläufern, die auf die erythroide Entwicklungsreihe festgelegt sind, aber noch große erythroide Kolonien (BFU-E) bilden können. Die Verunreinigung mit multipotenten Zellen und Zellen anderer Entwicklungsreihen beträgt weniger als 10 %.
ii) Der Effekt von Dexamethason, zusammen mit anderen Faktoren die Fähigkeit von menschlichen Proerythroblasten zur langandauernden Selbsterneuerung (mehr als 16 Zellteilungen) zu induzieren, spiegelt sich deutlich in den Kolonie- und Markeranalysen wider: sowohl die Fähigkeit, BFU-E (und BFU-E mix) zu bilden als auch die Fähigkeit, c-Kit zu exprimieren, wird durch Dexamethason entscheidend verstärkt.

### d) Regulation der Differenzierung der in vitro kultivierten Pro-Erythroblasten aus Nabelschnurblut: Anwendung der im Hühnersystem gewonnenen Ergebnisse.

Ein wesentlicher Vorteil normaler, zur Selbsterneuerung fähiger erythroider Vorläuferzellen im Hühnersystem war, daß die Zellen nach Entzug von "self renewal factors" (SCF, TGFα und Östradiol), und Ersatz dieser Faktoren durch Differenzierungsfaktoren (Epo, Insulin) mit normaler Kinetik und unter Durchlaufen der erwarteten Zahl von Zellteilungen zu reifen Erythrozyten ausdifferenzierten (Hayman et al., 1993). In Beispiel 7 wurde bereits gezeigt, daß diese Beobachtung prinzipiell auf menschliche Erythroblasten übertragbar war: menschliche, in vitro kultivierte Proerythroblasten reiften in rekombinantem humanem Epo und Insulin zu enukleierenden (den Kern ausstoßenden) Erythrozyten aus (Beispiel 7, Fig. 10A, Tafel A und B). Das Vorhandensein größerer Mengen von eindeutig zur Selbsterneuerung fähigen menschlichen Proerythroblasten gestattete es, diese Differenzierungsinduktion quantitativ zu untersuchen. Außerdem war es jetzt möglich, den Einfluß weiterer Faktoren auf das Differenzierungsprogramm der Zellen zu analysieren. Im Hühnersystem konnte gezeigt werden, daß SCF in Gegenwart von Epo und Insulin die erythroide Differenzierung stark verzögern bzw. während der ersten 4-5 Tage praktisch verhindern konnte (Hayman et al., 1993). Außerdem war das Schilddrüsenhormon T3 (Triiodo-Tyronin), vor allem zusammen mit Liganden des Co-Rezeptors RXR, in der Lage, die erythroide Differenzierung zu beschleunigen und die durch SCF bewirkte Verlangsamung der erythroiden Differenzierung komplett aufzuheben (Schroeder et al., 1992; Beug et al., 1994). Es war deshalb von Interesse, ob auch diese am Hühnersystem gewonnenen Beobachtungen auch für das menschliche System Gültigkeit haben. Während ein deutlicher, als Differenzierungsverzögerung deutbarer Effekt von SCF auf gereinigte menschliche BFU-E nachweisbar war (Dai et al., 1991; Sawada et al., 1991), sind direkte Untersuchungen zur Wirkung von T3 auf die Entwicklung gereinigter erythroider Vorläufer nicht bekannt.

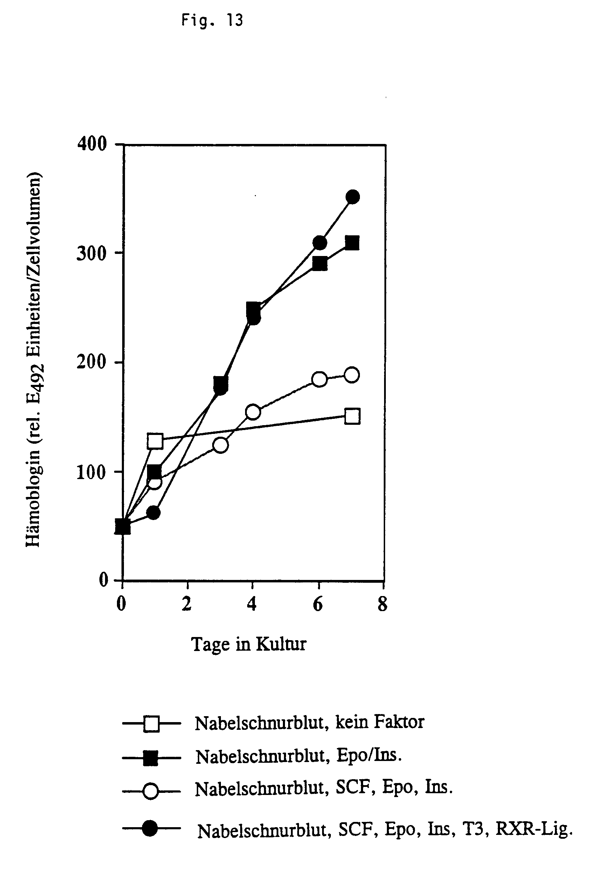

Die Versuche wurden mit 16 Tage alten Zellen aus der mit "factor mix" und Dexamethason gehaltenen Kultur durchgeführt. Die Zellen wurden abzentrifugiert, in Medium ohne Faktoren gewaschen und in einer Dichte von 1-:2x106 Zellen/ml in den verschiedenen Differenzierungsmedien kultiviert. Das Differenzierungsmedium enthielt 2 % humanes Serum (aus Nabelschnurblut) und entweder keine weiteren Zusätze (Fig. 13, weiße Quadrate; kein Faktor), 10 Einheiten/ml humanes rekombinantes Epo plus 10 ng/ml Insulin (Fig. 13, Epo, Ins; schwarze Quadrate), Epo, Ins plus 100 ng humaner SCF (Fig. 13, SCF, Epo, Ins; weiße Kreise) und die obigen Faktoren plus 200 nM Trijodothyronin und 10⁻⁶M 9 cis Retinolsäure (Fig. 13, SCF, Epo, Ins, T3, RXR Lig.; schwarze Kreise). Während der Differenzierung wurden die Zellen bei einer Dichte von 2-4 x 10⁶ Zellen/ml kultiviert und täglich frische Faktoren zugegeben. Zu den angegebenen Zeiten wurde das Zellvolumen in einem elektronischen Zellzähler des Typs CASY-1, Schärfe System, bestimmt (siehe Beispiel 7). Gleichzeitig wurde der Hämoglobingehalt von Zellaliquots bekannter Zellzahl durch photometrische Messung bestimmt (Kowenz et al., 1987). Die Ergebnisse sind in Fig. 13 dargestellt. Während, wie erwartet, in Abwesenheit von Epo/Ins der Hämoglobingehalt pro Zellvolumen kaum anstieg (Fig. 13, weiße Quadrate), war in Gegenwart von Epo/Insulin ein starker (ca. 8 facher) Anstieg des Hämoglobingehalts/Zellvolumen zu beobachten (Fig. 13, schwarze Quadrate). Überraschenderweise, verzögerte SCF die durch Epo/Insulin induzierte erythroide Differenzierung ganz ähnlich wie im Hühnersystem (Fig. 13, weiße Kreise), während Zusatz von Schilddrüsenhormon (T3) plus RXR-Ligand diese Verzögerung der Differenzierung durch SCF komplett aufhob ((Fig. 13, schwarze Kreise), wiederum exakt analog zu den im Hühnersystem erhaltenen Daten.

Zusammengefaßt zeigen diese Daten, daß menschliche, in Kultur zur Selbsterneuerung fähigen Proerythroblasten Erythropietin-abhängig in Kultur zu reifen, Hämoglobin akkumulierenden Erythrozyten ausreifen. Dieser Vorgang wird (wie in gereinigten menschlichen BFU-Es, Sawada et al., 1991) durch SCF verzögert und durch T3 beschleunigt.

**Tabelle I**

| Zelltyp | Kolonietyp (pro 10⁵ Zellen) | | |
|---|---|---|---|
| | BFU-E | BFU-mix | CFU-GM |
| 1 | 2250 | 60 | 70 |
| 2 | 5750 | 400 | 200 |
| 3 | 1200 | 45 | 115 |
| 4 | <1 | <1 | <1 |
| 5 | 2500 | 105 | 100 |
| 6 | ND | ND | ND |
| ND = nicht nachweisbar | | | |
| 1: Nabelschnurblut (13 Tage, Faktormischung) | | | |
| 2: Nabelschnurblut (13 Tage, Faktormischung + Dexamethason) | | | |
| 3: Nabelschnurblut (16 Tage, Faktormischung, unreife Fraktion <1.070 g/cm³) | | | |
| 4: Nabelschnurblut (16 Tage, Faktormischung, reife Fraktion >1.072 g/cm³) | | | |
| 5: Nabelschnurblut (16 Tage, Faktormischung + Dexamethason) | | | |
| 6: Peripheres Blut (CD34⁺, 9 Tage, Faktormischung + Dexamethason) | | | |

**Tabelle II**

| Zelltyp | Zelloberflächenmarker | | |
|---|---|---|---|
| | unreife | | erythroide |
| | CD71 (Transferrin-rezeptor) | CD117 (c-Kit,SCF-Rezeptor) | GPA (Glycophorin) |
| 1 | ND | ND | 66 % |
| 2 | ND | ND | 20 % |
| 3 | 88 % | 21 % | 9 % |
| 4 | 53 % | 21 % | 66 % |
| 5 | 84 % | 51 % | 7 % |
| 6 | 80 % | 65 % | 30 % |
| ND = nicht nachweisbar | | | |
| 1: Nabelschnurblut (13 Tage, Faktormischung) | | | |
| 2: Nabelschnurblut (13 Tage, Faktormischung + Dexamethason) | | | |
| 3: Nabelschnurblut (16 Tage, Faktormischung, unreife Fraktion <1.070 g/cm³) | | | |
| 4: Nabelschnurblut (16 Tage, Faktormischung, reife Fraktion >1.072 g/cm³) | | | |
| 5: Nabelschnurblut (16 Tage, Faktormischung + Dexamethason) | | | |
| 6: Peripheres Blut (CD34⁺, 9 Tage, Faktormischung + Dexamethason) | | | |

**Tabelle III**

| Zelltyp | Zelloberflächenmarker | |
|---|---|---|
| | nicht erythroide | |
| | CD33 (gran.) | CD3, CD19 (B-Zellen, T-Zellen) |
| 1 | 10 % | <0.1 % |
| 2 | 20 % | <0.1 % |
| 3 | 5 % | <0.1 % |
| 4 | <0.1 % | <0.1 % |
| 5 | 7 % | <0.1 % |
| 6 | 5 % | ND |
| ND = nicht nachweisbar | | |
| gran.=granulozytäre Zellen | | |
| 1: Nabelschnurblut (13 Tage, Faktormischung) | | |
| 2: Nabelschnurblut (13 Tage, Faktormischung + Dexamethason) | | |
| 3: Nabelschnurblut (16 Tage, Faktormischung, unreife Fraktion <1.070 g/cm³) | | |
| 4: Nabelschnurblut (16 Tage, Faktormischung, reife Fraktion >1.072 g/cm³) | | |
| 5: Nabelschnurblut (16 Tage, Faktormischung + Dexamethason) | | |
| 6: Peripheres Blut (CD34⁺, 9 Tage, Faktormischung + Dexamethason) | | |

### Literatur

Beug, H., Palmieri, S., Freudenstein, C., Zentgraf, H. und Graf, T., 1982, Cell 28, 907.
Beug, H., Döderlein, G. und Zenke, M., 1992, in Nuclear Processes and Oncogenes, (P.A. Sharp, ed.) p.53, Academic Press Inc., Harcourt Brace Jovanovich, Publishers San Diego.
Beug, H., Müllner, E. W., and Hayman, M. J., 1994. Curr. Op. Cell Biol. 6, 816-824.
Boulay, J.L. und Paul, W.E., 1993, Current Biology 3, 573-581.
Dai, C. H., Krantz, S. B., and Zsebo, K. M., 1991. Blood 78, 2493-2497.
Daley, G.Q., Van Elten, R.A. und Baltimore, D., 1990, Science 247, 824-830.
Elefanty, A.G., Hariharan, I.K. und Cory, S., 1990, EMBO J. 9, 1069-1078.
Fantl, W.J. et al., 1993, Annual Reviews of Biochemistry 62, 453-481.
Galimi, F., Bagnara, G.P., Bonsi, L., Cottone, E., Follenzi, A., Simeone, A. und Comoglio, P.M., 1994, J. Cell. Biol. 127, 1743-1754.
Graf, T. und Beug, H., 1978, Biochim. Biophys. Acta 516, 269-299.
Hayman, M.J., Meyer, S., Martin, F., Steinlein, P. und Beug, H., 1993, Cell 74, 157-169.
Hogan, B., 1993, Current Biology 170-172.
Jolly, D., 1994, Cancer Gene Therapy 1, 51.
Kaipainen, A., Korhonen, J., Pajusola, K., Aprelikova, O., Persico, M.G., Terman, B.I. und Alitalo, K., 1993, J. Exp. Med. 178, 2077-2088.
Keller, G., 1992, Curr. Opinion in Immunology 4, 133-139.
Kelliher, M.A., McLaughlin, J., Witte, O.N. und Rosenberg, N., 1990, Proc.Natl.Acad.Sci. USA 87, 6649-6653.
Kowenz, E., Leutz, A., Doderlein, G., Graf, T., and Beug, H., 1987. In Modern Trends in Human Leukemia VII, R. Neth, R. C. Gallo, M. F. Greaves and H. Kabisch, eds. (Heidelberg: Springer Verlag), pp. 199-209.
Laufer, E., 1993, Current Biology 3, 306-308.
Lax, I., Johnson, A., Howk, R., Sap, J., Bellot, F., Winkler, M., Ullrich, A., Vennström, B., Schlessinger, J. und Givol, D., 1988, Mol. Cell. Biol. 8, 1970-1978.
Leutz, A., Beug, H. und Graf, T., 1984, EMBO J. 3, 3191-3197.
Metcalf, D., 1980, Proc.Natl.Acad.Sci. USA 77, 5327-5330.
Mitani, K. und Caskey, C.T., 1993, Trends in Biotechnology 11, 162-166.
Peles, E. und Yarden, Y., 1993, Bioessays 15, 815-824.
Rolink, A., Kudo, A., Karasyama, H. und Kikuchi, Y., 1991, EMBO J. 10, 327-336.
Sawada, K., Krantz, S. B., Dai, C. H., Koury, S. T., Horn, S. T., Glick, A. D., and Civin, C. I., 1990. J. Cell. Physiol. 142, 219-230.
Sawada, K., Krantz, S. B., Dai, C. H., Sato, N., Ieko, M., Sakurama, S., Yasukouchi, T., and Nakagawa, S., 1991. J. Cell. Physiol. 149, 1-8.
Sawada K., et al., J. Cell. Physiol.142, 219-230
Sawyers, C.L., Denny, C.T. und Witte, O.N., 1991, Cell 64, 337-350.
Schroeder, C., Gibson, L., Zenke, M. and Beug, H., 1992, Oncogene 7, 217-227.
Schroeder, C., Gibson, L., Nordström, Ch. und Beug, H., 1993, EMBO J. 12, 951-960.
Shpall, E.J. Jones, R.B., Bearman, S.I., Franklin, W.A., Archer, P.G., Curiel, T., Bitter, M. Claman, H.N. Stemmer, S.M., Purdy, M., Myers, S.E., Hami, L., Taffs, S., Heimfeld, S., Hallagan, J., Berenson, R.J., 1994, J. Clin. Oncol. 12, 28-36.
Tamagnone, L., Partanen, J., Armstrong, E., Lasota, J., Ohgami, K., Tazunoki, T., LaForgia, S., Huebner, K. und Alitalo, K., 1993, Oncogene 8, 2009-2014.
Tepper, R.I. und Mule, J.J., 1994, Human Gene Therapy 5, 153.
Till, J.E. und McCulloch, M., 1980, Biochim. Biophys. Acta 605, 431-459.
Van der Geer, P., Hunter, T. und Lindberg, R.A., 1994, Annual Review Cell Biol. 10, 251-337.
Vile, R. und Russel S., 1994, Gene Therapy 1, 88.
Zatloukal, K., Schmidt, W., Cotten, M., Wagner, E., Stingl, G. und Birnstiel, M.L., 1993, Gene 135, 199.
Hematopoietic Stem Cells, The Mulhouse Manual, 1994, Hsg. Wunder, E., Sovalat, H., Henon, P.R. und Serke, S.

## Patentansprüche

1. Verfahren zur *in vitro* Herstellung von nicht-immortalisierten hämatopoetischen Vorläuferzellen der erythroiden Entwicklungsreihe, **dadurch gekennzeichnet, daß** man Zellen, enthaltend eine Population erythroider Vorläufer, in einem Medium, das die für das Wachstum erythroider Zellen erforderlichen üblichen Komponenten enthält, zumindest solange mit einer Kombination aus Wachstumsfaktoren, enthaltend
a) mindestens einen Liganden des Östrogenrezeptors, ausgewählt aus natürlichen oder synthetischen Steroidhormonen, die den Östrogenrezeptor wie Östradiol aktivieren,
b) mindestens einen Liganden des Glucocorticoid-Rezeptors, ausgewählt aus natürlichen oder synthetischen Steroidhormonen, die den Glucocorticoidrezeptor wie Hydrocortison aktivieren, und
c) mindestens einen Liganden eines Tyrosinkinase-Rezeptors, der diesen Rezeptor aktiviert, behandelt, bis die Zellen sich selbst zu erneuern beginnen, und daß man gegebenenfalls anschließend die Zellen in einem Medium weiterzüchtet, welches die für die anhaltende Selbsterneuerung erforderlichen Faktoren enthält.

2. Verfahren nach Anspruch 1, **dadurch gekennzeichnet, daß** die Zellen humane Zellen sind.

3. Verfahren nach Anspruch 2, **dadurch gekennzeichnet, daß** als Ausgangszellen eine auf CD34-positive Zellen angereicherte Zellpopulation verwendet wird.

4. Verfahren nach Anspruch 3, **dadurch gekennzeichnet, daß** eine Zellpopulation aus Knochenmark, peripherem Blut oder aus Nabelschnurblut verwendet wird.

5. Verfahren nach einem der vorhergehenden Ansprüche, **dadurch gekennzeichnet, daß** die Faktorkombination mindestens zwei Liganden von Tyrosinkinase-Rezeptoren enthält.

6. Verfahren nach Anspruch 5, **dadurch gekennzeichnet, daß** die Faktorkombination mindestens zwei Liganden enthält, die an Rezeptoren aus verschiedenen Klassen von Tyrosinkinase-Rezeptoren binden.

7. Verfahren nach Anspruch 6, **dadurch gekennzeichnet, daß** die Liganden ausgewählt sind aus Gruppen, die an Rezeptoren mit unterschiedlich strukturierter Kinasedomäne binden.

8. Verfahren nach Anspruch 7, **dadurch gekennzeichnet, daß** die Liganden ausgewählt sind aus den Gruppen
i) Liganden, die an Tyrosinkinase-Rezeptoren binden, die eine durchgehende Kinasedomäne besitzen; und
ii) Liganden, die an Tyrosinkinase-Rezeptoren binden, die eine von einem Insert unterbrochene Kinasedomäne besitzen.

9. Verfahren nach Anspruch 8, **dadurch gekennzeichnet, daß** die Faktorkombination
i) einen Liganden eines Rezeptors aus der Familie der Epidermaler-Wachstumsfakor (EGF)-Rezeptoren und/oder des Hepatozyten-Wachstumsfaktor (HGF)-Rezeptors und
ii) einen Liganden von c-Kit
enthält.

10. Verfahren nach Anspruch 9, **dadurch gekennzeichnet, daß** die Faktorkombination
i) Transformierender Wachstumsfaktor α (TGFα) und/oder Epidermaler Wachstumsfaktor (EGF) und/oder Hepatozytenwachstumsfaktor (HGF),
ii) stammzellfaktor (SCF)
iii) Dexamethason und/oder Hydrocortison, und Östradiol
enthält.

11. Verfahren nach einem der vorhergehenden Ansprüche, **dadurch gekennzeichnet, daß** die Faktorenkombination außerdem einen oder mehrere weitere Faktoren enthält, die die Erlangung des Selbsterneuerungspotentials beschleunigen.

12. Verfahren nach Anspruch 11, **dadurch gekennzeichnet, daß** der bzw. die weitere(n) Faktoren ausgewählt sind aus den Gruppen Zytokine und/oder Liganden von Tyrosinkinase- und/oder Serinkinase-Rezeptoren.

13. Verfahren nach Anspruch 12, **dadurch gekennzeichnet, daß** das Zytokin Erythropoetin ist.

14. Verfahren nach Anspruch 12, **dadurch gekennzeichnet, daß** der Tyrosinkinase-Rezeptorligand Insulin-artiger Wachstumsfaktor-1 (IGF-1) ist.

15. Verfahren nach einem der Ansprüche 10 bis 14, **dadurch gekennzeichnet, daß** die Faktorkombination
i) Transformierender Wachstumsfaktor α (TGFα) und/oder Epidermaler Wachstumsfaktor (EGF) und/oder Hepatozytenwachstumsfaktor (HGF),
ii) Stammzellfaktor (SCF),
iii) Dexamethason und Östradiol,
iv) Erythropoetin und (IGF-1) enthält.

16. Verfahren nach einem der vorhergehenden Ansprüche, **dadurch gekennzeichnet, daß** man die Zellen nach Erlangung des Selbsterneuerungspotentials in Gegenwart von Faktoren, die für die anhaltende Selbsterneuerung der Zellen erforderlich sind, weiterzüchtet.

17. Verfahren nach Anspruch 16, daß man humane Zellen in Gegenwart mindestens eines Liganden der Familie der EGF-Rezeptoren oder des HGF-Rezeptors, SCF, Erythropoetin und IGF-1 weiterzüchtet.

18. Verfahren nach Anspruch 17, **dadurch gekennzeichnet, daß** der Ligand EGF und/oder TGFα und/oder HGF ist.

## Claims

1. Process for *in vitro* production of non-immortalised haematopoietic progenitor cells of the erythroid lineage, which is **characterised in that** cells containing a population of erythroid progenitors, in a medium which contains the usual components necessary for the growth of erythroid cells, are treated with a combination of growth factors, containing
a) at least one ligand of the oestrogen receptor, selected from natural or synthetic steroid hormones, which activate the oestrogen receptor like oestradiol,
b) at least one ligand of the glucocorticoid receptor selected from natural or synthetic steroid hormones, which activate the glucocorticoid receptor like hydrocortisone, and
c) at least one ligand of a tyrosine-kinase receptor which activates this receptor,
at least until the cells begin to renew themselves, and subsequently, if desired, the cells are further cultured in a medium which contains the factors required for sustained self-renewal.

2. Process according to claim 1, **characterised in that** the cells are human cells.

3. Process according to claim 2, **characterised in that** the starting cells used are a cell population with a concentration of CD34-positive cells.

4. Process according to claim 3, **characterised in that** a cell population from bone marrow, peripheral blood or umbilical cord blood is used.

5. Process according to one of the preceding claims, **characterised in that** the factor combination contains at least two ligands of tyrosine kinase receptors.

6. Process according to claim 5, **characterised in that** the factor combination contains at least two ligands which bind to receptors from different classes of tyrosine kinase receptors.

7. Process according to claim 6, **characterised in that** the ligands are selected from groups which bind to receptors with differently structured kinase domains.

8. Process according to claim 7, **characterised in that** the ligands are selected from the groups
i) ligands which bind to tyrosine kinase receptors, which have a continuous kinase domain; and
ii) ligands which bind to tyrosine kinase receptors which have a kinase domain interrupted by an insert.

9. Process according to claim 8, **characterised in that** the factor combination contains
i) a ligand of a receptor from the family of the epidermal growth factor (EGF) receptors and/or the hepatocyte growth factor (HGF) receptor and
ii) a ligand of c-Kit.

10. Process according to claim 9, **characterised in that** the factor combination contains
i) transforming growth factor α (TGFα) and/or epidermal growth factor (EGF) and/or hepatocyte growth factor (HGF),
ii) stem cell factor (SCF),
iii) dexamethasone and/or hydrocortisone, and oestradiol.

11. Process according to one of the preceding claims, **characterised in that** the factor combination also contains one or more other factors which accelerate the acquisition of the self-renewal potential.

12. Process according to claim 11, **characterised in that** the additional factor(s) is or are selected from the groups comprising the cytokines and/or ligands of tyrosine kinase and/or serine kinase receptors.

13. Process according to claim 12, **characterised in that** the cytokine is erythropoietin.

14. Process according to claim 12, **characterised in that** the tyrosine kinase receptor ligand is insulin-like growth factor (IGF-1).

15. Process according to one of claims 10 to 14, **characterised in that** the factor combination contains
i) transforming growth factor α (TGFα) and/or epidermal growth factor (EGF) and/or hepatocyte growth factor (HGF),
ii) stem cell factor (SCF),
iii) dexamethasone and oestradiol,
iv) erythropoietin and IGF-1.

16. Process according to one of the preceding claims, **characterised in that** the cells are further cultivated after the self-renewal potential has been acquired in the presence of factors which are necessary for sustained self-renewal of the cells.

17. Process according to claim 16, **characterised in that** human cells are further cultivated in the presence of at least one ligand of the family of the EGF receptors or the HGF receptor, SCF, erythropoietin and IGF-1.

18. Process according to claim 17, **characterised in that** the ligand is EGF and/or TGFα and/or HGF.

## Revendications

1. Procédé pour la fabrication *in vitro* de cellules précurseurs hématopoïétiques non immortalisées de la série de développement érythroïde, **caractérisé en ce que** l'on traite des cellules contenant une population de précurseurs érythroïdes dans un milieu qui contient les composants usuels pour la croissance de cellules érythroïdes avec une combinaison de facteurs de croissance contenant
a) au moins un ligand du récepteur des oestrogènes, choisi parmi des hormones stéroïdes naturelles ou synthétiques qui activent le récepteur des oestrogènes, par exemple l'oestradiol,
b) au moins un ligand du récepteur des glucocorticoïdes, choisi parmi des hormones stéroïdes naturelles ou synthétiques qui activent le récepteur des glucocorticoïdes, par exemple l'hydrocortisone, et
c) au moins un ligand d'un récepteur de la tyrosine kinase qui active ce récepteur,
au moins jusqu'à ce que les cellules commencent à se régénérer d'elles-mêmes, et **en ce que** l'on continue le cas échéant à cultiver les cellules dans un milieu qui contient les facteurs nécessaires pour entretenir l'auto-régénération.

2. Procédé selon la revendication 1, **caractérisé en ce que** les cellules sont des cellules humaines.

3. Procédé selon la revendication 2, **caractérisé en ce que** l'on utilise comme cellules de départ une population cellulaire enrichie en cellules positives pour le CD34.

4. Procédé selon la revendication 3, **caractérisé en ce que** l'on utilise une population cellulaire provenant de la moelle osseuse, du sang périphérique ou du sang ombilical.

5. Procédé selon l'une des revendications précédentes, **caractérisé en ce que** la combinaison de facteurs contient au moins deux ligands de récepteurs de la tyrosine kinase.

6. Procédé selon la revendication 5, **caractérisé en ce que** la combinaison de facteurs contient au moins deux ligands qui se lient à des récepteurs provenant de deux classes différentes de récepteurs de la tyrosine kinase.

7. Procédé selon la revendication 6, **caractérisé en ce que** les ligands sont choisis dans des groupes comprenant des récepteurs qui se lient à des domaines de la kinase ayant des structures différentes.

8. Procédé selon la revendication 7, **caractérisé en ce que** les ligands sont choisis dans les groupes comprenant
i) des ligands qui se lient à des récepteurs de la tyrosine kinase qui possèdent un domaine de la kinase ininterrompu et
ii) des ligands qui se lient à des récepteurs de la tyrosine kinase qui possèdent un domaine de la kinase interrompu par un segment d'insertion.

9. Procédé selon la revendication 8, **caractérisé en ce que** la combinaison de facteurs contient
i) un ligand d'un récepteur provenant de la famille comprenant les récepteurs du facteur de croissance épidermique (EGF) et/ou du récepteur du facteur de croissance des hépatocytes (HGF) et
ii) un ligand du kit c.

10. Procédé selon la revendication 9, **caractérisé en ce que** la combinaison de facteurs contient
i) du facteur de croissance transformant α (TGFα) et/ou du facteur de croissance épidermique (EGF) et/ou du facteur de croissance des hépatocytes (HGF),
ii) du facteur de cellule souche (SCF)
iii) de la dexaméthasone et/ou de l'hydrocortisone et de l'oestradiol

11. Procédé selon l'une des revendications précédentes, **caractérisé en ce que** la combinaison de facteurs contient en plus un ou plusieurs autres facteurs qui accélèrent l'atteinte du potentiel d'auto-régénération.

12. Procédé selon la revendication 11, **caractérisé en ce que** le ou les autres facteurs sont choisis dans le groupe comprenant des cytokines et/ou des ligands des récepteurs de la tyrosine kinase et/ou des récepteurs de la sérine kinase.

13. Procédé selon la revendication 12, **caractérisé en ce que** la cytokine est l'érythropoïétine.

14. Procédé selon la revendication 12, **caractérisé en ce que** le ligand du récepteur de la tyrosine kinase est un facteur de croissance 1 analogue à l'insuline (IGF-1).

15. Procédé selon l'une des revendications 10 à 14, **caractérisé en ce que** la combinaison de facteurs contient
i) du facteur de croissance transformant α (TGFα) et/ou du facteur de croissance épidermique (EGF) et/ou du facteur de croissance des hépatocytes (HGF),
ii) du facteur de cellule souche (SCF),
iii) de la dexaméthasone et de l'oestradiol,
iv) de l'érythropoïétine et de l'IGF-1.

16. Procédé selon l'une des revendications précédentes, **caractérisé en ce que**, une fois atteint le potentiel d'auto-régénération, l'on continue à cultiver les cellules en présence de facteurs qui sont nécessaires pour entretenir l'auto-régénération des cellules.

17. Procédé selon la revendication 16, **caractérisé en ce que** l'on continue à cultiver des cellules humaines en présence d'au moins un ligand de la famille des récepteurs de l'EGF ou du récepteur du HGF, du SCF, de l'érythropoïétine et de l'IGF-1.

18. Procédé selon la revendication 17, **caractérisé en ce que** le ligand est l'EGF et/ou le TGFα et/ou le HGF.
